(19) Europäisches Patentamt
European Patent Office
Office européen des brevets

(11) Veröffentlichungsnummer: **0 033 504**
**B1**

(12) # EUROPÄISCHE PATENTSCHRIFT

(45) Veröffentlichungstag der Patentschrift: **24.10.84**

(51) Int. Cl.³: **C 07 C 103/52,** C 07 F 9/38, C 07 C 143/14, A 61 K 37/02

(21) Anmeldenummer: **81100545.3**

(22) Anmeldetag: **26.01.81**

(54) **Pentapeptide vom Encephalintyp, Verfahren zur Herstellung derselben und diese enthaltende Arzneimittel.**

(30) Priorität: **25.01.80 HU RI000155**

(43) Veröffentlichungstag der Anmeldung: **12.08.81 Patentblatt 81/32**

(45) Bekanntmachung des Hinweises auf die Patenterteilung: **24.10.84 Patentblatt 84/43**

(84) Benannte Vertragsstaaten: **BE CH DE FR GB IT LI NL**

(56) Entgegenhaltungen: **DE - A - 2 740 699**

**Chemical Abstracts Band 94, Nr. 5, 2. Februar 1981 Columbus, Ohio, USA S. BAJUSZ et al. "Enkephalin analogs containing amino sulfonic acid and amino phosphonic acid residues at position 5" Seite 93, Spalte 2, Abstract Nr. 25410n**

(73) Patentinhaber: **Richter Gedeon Vegyészeti Gyár R.T., Gyömröi ut 19-21, H-1475 Budapest X (HU)**

(72) Erfinder: **Bajusz, Sándor, Dr., Derék u. 16/a, Budapest I. (HU)**
Erfinder: **Rónai, András, Dr., Halmi u. 2, Budapest XI (HU)**
Erfinder: **Székely, József, Dr., Rudas László u. 43, Budapest VI (HU)**

(74) Vertreter: **Beszédes, Stephan G. Dr., Münchener Strasse 80a Postfach 1168, D-8060 Dachau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue Pentapeptide vom Encephalintyp, ein Verfahren zu ihrer Herstellung und diese enthaltende Arzneimittel, insbesondere solche mit Wirkungen auf das Zentralnervensystem, vor allem Opiatwirksamkeit beziehungsweise morphinartiger Wirkung.

Es ist bekannt, dass die morphinartige Wirkung beziehungsweise Opiataktivität des aus dem Gehirn isolierten Methionin- beziehungsweise Leucinencephalines, das heisst der beiden Pentapeptide L-Tyr$\overset{1}{o}$syl-gly$\overset{2}{c}$yl-gly$\overset{3}{c}$yl-L-phen$\overset{4}{y}$lalanyl-L-meth$\overset{5}{i}$onin beziehungsweise L-Tyr$\overset{1}{o}$syl-gly$\overset{2}{c}$yl-gly$\overset{3}{c}$yl-L-phen$\overset{4}{y}$lalanyl-L-leu$\overset{5}{c}$in (Hughes und Mitarbeiter: Nature 258 [1975], 577) durch Ersatz bestimmter Aminosäurereste derselben, in erster Linie der Einheiten Gly$\overset{2}{c}$yl und Meth$\overset{5}{i}$onin beziehungsweise Leu$\overset{5}{c}$in, durch andere Aminosäurereste gesteigert werden kann. Als Beispiele seien die Pentapeptide vom Encephalintyp der folgenden Strukturen erwähnt: L-Tyrosyl-D-methionyl-glycyl-L-phenylalanyl-L-prolinamid (belgische Patentschrift 858 453), L-Tyrosyl-D-alanyl-glycyl-(N-methyl)-L-phenylalanyl-L-methionin-ol-S-sulfoxyd (Roemer und Mitarbeiter: Nature 268 [1977] 547) und L-Tyrosyl-D-alanyl-glycyl-L-phenylalanyl-D-leucin (Baxter und Mitarbeiter: Br. J. Pharmacol. 59 [1977], 455).

Beim Vergleich der am Krummdarm beziehungsweise Ileum von Meerschweinchen (GPI [guinea-pig ileum]) und an Maussamenleiterpräparaten (MVD [mouse vas deferens]) gemessenen Wirksamkeiten der opioiden Peptide natürlichen Ursprunges einerseits und des Morphines beziehungsweise Normorphines anderseits ist festzustellen, dass die Opiatwirkung dieser Verbindungen von verschiedenem Charakter ist. Für die Encephaline ist es kennzeichnend, dass sie an Maussamenleiterpräparaten wesentlich wirksamer sind als am Krummdarm von Meerschweinchen, während das Morphin am Krummdarm von Meerschweinchen die grössere Wirksamkeit zeigt. Das natürlich vorkommende β-Endorphin (ein Polypeptid mit 31 Gliedern) hat am Krummdarm von Merschweinchen und an Maussamenleiterpräparaten die gleiche Wirksamkeit, was darauf schliessen lässt, dass es mit den in den Präparaten befindlichen Rezeptoren in der gleichen Weise in Wechselwirkung tritt. Von den erwähnten durch Abwandlung erhaltenen Pentapeptiden vom Encephalintyp ist das L-Tyrosyl-D-alanyl-glycyl-L-phenylalanyl-D-leucin an Maussamenleiterpräparaten wirksamer als am Krummdarm von Meerschweinchen, der Charakter seiner Opiatwirkung ist also dem der der Encephaline ähnlich. Die Wirksamkeit des L-Tyrosyl-D-methionyl-glycyl-L-phenylalanyl-L-prolinamides ist in beiden Versuchen die gleiche, während das L-Tyrosyl-D-alanyl-glycyl-(N-methyl)-L-phenylalanyl-L-methionin-ol-S-sulfoxyd am Krummdarm von Meerschweinchen doppelt so wirksam ist wie an Maussamenleiterpräparaten. Demnach ähnelt die Opiatwirkung der beiden letztgenannten Verbindungen eher der des β-Endorphines beziehungsweise Morphines als der der Encephaline. Ferner sind aus dem Schrifttum einige Dipeptide, welche an Stelle der endständigen Carboxylgruppe eine Phosphonsäuregruppe (Gilemor und McBridge: J. Pharm. Sci. 63 [1974], 1087) beziehungsweise Sulfonsäuregruppe (Shiba und Mitarbeiter: Bull. Chem. Soc. Japan 50 [1977], 254) aufweisen, bekannt. Für diese Peptidderivate ist es charakteristisch, dass die Dissoziationskonstante ihrer Säuregruppe grösser ist als die der Carboxylgruppe der echten Peptide beziehungsweise dass die Phosphonsäurederivate noch eine zweite Hydroxygruppe, deren Dissoziationskonstante geringer als die der Carboxylgruppe ist, aufweisen.

Der Erfindung liegt die Aufgabe zugrunde, neue Pentapeptide vom Encephalintyp die von den bekannten vor allem an der Stelle des endständigen Kohlenstoffatomes abweichen, mit überlegenen pharmakologischen Wirkungen, insbesondere solchen auf das Zentralnervensystem, vor allem grösserer morphinartiger Wirkung beziehungsweise Opiatwirksamkeit, ein Verfahren zur Herstellung derselben und diese Verbindungen enthaltende Arzneimittel zu schaffen.

Das Obige wurde überraschenderweise durch die Erfindung erreicht.

Es wurde nämlich überraschenderweise festgestellt, dass insbesondere die Opiatwirksamkeit von Pentapeptiden vom Encephalintyp, bei welchen an Stelle der endständigen Carboxylgruppe eine endständige Phosphonsäuregruppe oder Sulfonsäuregruppe ist, erhöht ist.

Gegenstand der Erfindung sind daher Pentapeptide vom Encephalintyp der allgemeinen Formel

$$\text{Tyr–X–Gly–Phe–Y} \qquad \text{I,}$$

worin

Tyr einen L-Tyrosylrest,

Gly einen Rest von Glykokoll,

Phe einen Rest von L-Phenylalanin,

X einen Rest von Glykokoll oder einen Rest einer D-α-Aminocarbonsäure, welcher eine Alkylseitenkette mit 1 bis 4 Kohlenstoffatom(en), eine, gegebenenfalls S-methylsubstituierte, Thioalkylseitenkette mit 1 bis 4 Kohlenstoffatom(en) oder eine Phenylalkylseitenkette mit 1 bis 4 Kohlenstoffatom(en) im Alkylteil aufweist und

Y einen Rest einer L-, D- oder DL-α-Aminophosphonsäure oder L-, D- oder DL-α-Aminosulfonsäure, welcher eine Alkylenseitenkette mit 1 bis 4 Kohlenstoffatom(en) aufweist, darstellen, sowie ihre Salze.

In diesem Text ist der Begriff «Pentapeptid» in seinem weitesten Sinne als «Peptid» mit 5 Aminosäureresteinheiten, von denen nicht alle Aminocarbonsäureresteinheiten zu sein brauchen, in der Peptidkette aufzufassen.

Die Bezeichnungen Tyr, Gly und Phe sind im Fachschrifttum (zum Beispiel J. Biol. Chem. 247

[1972], 977) zur Kennzeichnung von Aminosäuren üblich.

Vorzugsweise ist die Alkylseitenkette beziehungsweise Thioalkylseitenkette des Restes der D-α-Aminocarbonsäure, für den X stehen kann, eine solche mit 1, 2 oder 4 Kohlenstoffatom(en).

Es ist auch bevorzugt, dass die Phenylalkylseitenkette des Restes der D-α-Aminocarbonsäure, für den X stehen kann, eine solche mit 1 oder 2, insbesondere 1, Kohlenstoffatom(en) im Alkylteil ist.

Ferner ist es bevorzugt, dass der Rest der D-α-Aminocarbonsäure, für den X stehen kann, ein Rest von D-Alanin, D-Norleucin, D-Methionin oder D-Phenylalanin ist.

Es ist bevorzugt, dass der Rest der D-α-Aminocarbonsäure, für den X stehen kann, ein Rest von D-Alanin, D-Norleucin, D-Methionin oder D-Phenylalanin ist.

Der Rest, für den Y steht, kann geradkettig oder verzweigt sein.

Bevorzugt sind die Reste, für die Y steht, in der L- oder DL-Konfiguration.

Besonders bevorzugte erfindungsgemässe Verbindungen sind L-Tyrosyl-glycyl-glycyl-L-phenylalanyl-L-α-aminopentanphosphonsäure, L-Tyrosyl-D-alanyl-glycyl-L-phenylalanyl-L-α-aminopentanphosphonsäure, L-Tyrosyl-D-alanyl-glycyl-L-phenylalanyl-DL-α-aminopentansulfonsäure, L-Tyrosyl-D-methionyl-glycyl-L-phenylalanyl-L-α-aminopentanphosphonsäure, L-Tyrosyl-D-norleucyl-glycyl-L-phenylalanyl-L-α-aminopentanphosphonsäure und L-Tyrosyl-D-norleucyl-glycyl-L-phenylalanyl-L-α-aminopentansulfonsäure.

Von den Salzen der erfindungsgemässen Peptide sind die Zink(II)- und Kupfer(II)-salze bevorzugt.

Gegenstand der Erfindung ist auch ein Verfahren zur Herstellung der erfindungsgemässen Verbindungen, welches dadurch gekennzeichnet ist, dass in der Peptidchemie an sich bekannter Weise eine L-, D- oder DL-α-Aminophosphonsäure oder L-, D- oder DL-α-Aminosulfonsäure mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat derselben kondensiert wird und gegebenenfalls mit dem erhaltenen am endständigen Stickstoffatom geschützten Peptidzwischenprodukt und dem beziehungsweise den durch etwaige weitere derartige Kondensationen erhaltenen am endständigen Stickstoffatom geschützten weiteren Peptidzwischenprodukt(en) nach in an sich bekannter Weise erfolgendem Entfernen der Schutzgruppe des endständigen Stickstoffatomes eine weitere beziehungsweise weitere Kondensation(en) mit beziehungsweise jeweils mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat derselben vorgenommen wird, wobei so viele Kondensationen durchgeführt werden, wie es zum Einbau aller gewünschten Aminosäureeinheiten erforderlich ist, sowie danach in an sich bekannter Weise vom erhaltenen geschützten Pentapeptidderivat die Schutzgruppe des endständigen Stickstoffatomes und die etwaige(n) Schutzgruppe(n) von anderen Stickstoffatomen beziehungsweise Sauerstoffatomen entfernt wird beziehungsweise werden, worauf gegebenenfalls in an sich bekannter Weise das erhaltene Pentapeptid der allgemeinen Formel in ein Säureadditionssalz desselben überführt wird beziehungsweise gegebenenfalls das erhaltene Säureadditionssalz des Pentapeptides der allgemeinen Formel in ein anderes Säureadditionssalz oder in das Pentapeptid der allgemeinen Formel überführt wird.

Gemäss einer bevorzugten Ausführungsform des erfindungsgemässen Verfahrens wird in der Weise vorgegangen, dass eine DL-α-Aminophosphonsäure oder -sulfonsäure mit einer Alkylseitenkette mit 4 Kohlenstoffatomen in Gegenwart einer äquivalenten Menge Base mit einem aus an der Aminogruppe geschütztem L-Phenylalanin erhaltenen gemischten Anhydrid acyliert sind, nach dem Entfernen der Schutzgruppe die erhaltenen freien Dipeptid-Diastereomere in die L-L- und L-D-Dipeptide getrennt werden und unter Anwendung der Verfahrensweise der gemischten Anhydride oder der Aktivesterverfahrensweise das L-L- oder L-D-Dipeptid mit einem an der endständigen Aminogruppe eine abspaltbare Schutzgruppe, vorzugsweise tert.-Butyloxycarbonylgruppe, aufweisenden Tripeptidfragment gekoppelt wird sowie vom erhaltenen geschützten Pentapeptid die Schutzgruppe abgespalten und das freie Pentapeptid oder gegebenenfalls sein Salz isoliert wird.

Ferner sind erfindungsgemäss Arzneimittel welche 1 oder mehr der erfindungsgemässen Verbindungen als Wirkstoff beziehungsweise Wirkstoffe, zweckmässigerweise zusammen mit üblichen pharmazeutischen Konfektionierungsmitteln, enthalten, vorgesehen.

Die erfindungsgemässen Verbindungen haben nämlich wie bereits erwähnt wertvolle pharmakologische Wirkungen, insbesondere solche auf das Zentralnervensystem, vor allem eine überlegene Opiatwirksamkeit.

So ist beziehungsweise sind die an Maussamenleiterpräparaten und die am Krummdarm von Merschweinchen gemessene(n) Opiatwirksamkeit(en) und/oder der Quotient dieser beiden Aktivitäten (der für die Encephaline charakteristische höhere Quotient

$$\text{von}\ \frac{\text{Opiatwirksamkeit an Maussamenleiterpräparaten}}{\text{Opiatwirksamkeit am Krummdarm von Meerschweinchen}}\ \text{[MVD/GPI-Protenzquotient]}$$

bei den erfindungsgemässen Verbindungen überraschenderweise höher als die bei den entsprechenden Verbindungen, welche die gleiche Aminosäurereihenfolge, jedoch endständig eine Carboxylgruppe aufweisen, und eine morphinartige Wirkung haben. Besonders gross ist der Unterschied, wenn der Rest, für den Y steht, in der L- oder DL-Konfiguration ist.

Zur Veranschaulichung dieses Unterschiedes sind in der folgenden Tabelle I die an Maussamenleiterpräparaten (Hughes und Mitarbeiter: Br. J. Pharmacol. 53 [1975], 371) und am Krummdarm von Meerschweinchen (Kosterlitz und Mitarbeiter: Br. J. Pharmacol. 39 [1970], 398) gemessenen relativen Opiatwirksamkeiten {die am Maussamenleiterpräparat beziehungsweise am Krummdarm von Meerschweinchen erhaltenen Werte sind auf die am Krummdarm von Meerschweinchen gemessene Wirksamkeit des L-Tyrosyl-glycyl-glycyl-L-phenylalanyl-L-methionines [Met-Encephalines] ($ID_{50}$-Wert = 183,5 nM) als Einheit bezogen} von erfindungsgemässen Verbindungen der allgemeinen Formel I und der ihnen entsprechenden bekannten Verbindungen, welche sich von den ersteren durch ihre endständige Carboxylgruppe oder anders ausgedrückt endständige Aminosäureeinheit unterscheiden, zusammengestellt.

Tabelle I

| Verbindung | Opiatwirksamkeit an Maussamenleiterpräparaten | Opiatwirksamkeit am Krummdarm von Meerschweinchen | Opiatwirksamkeit an Maussamenleiterpräparaten / Opiatwirksamkeit am Krummdarm von Meerschweinchen |
|---|---|---|---|
| L-Tyrosyl-glycyl-glycyl-L-phenylalanyl-L-α-aminopentanphosphonsäure [erfindungsgemäss] | 82,3 | 1,4 | 58,8 |
| L-Tyrosyl-glycyl-glycyl-L-phenylalanyl-L-α-aminopentansulfonsäure [erfindungsgemäss] | 43,7 | 3,1 | 14,1 |
| L-Tyrosyl-glycyl-glycyl-L-phenylalanyl-L-phenylalanyl-L-norleucin* [Vergleichssubstanz] | 8,9 | 0,5 | 17,8 |
| L-Tyrosyl-D-alanyl-glycyl-L-phenylalanyl-L-α-aminopentanphosphonsäure [erfindungsgemäss] | 941,0 | 11,2 | 84,0 |
| L-Tyrosyl-D-alanyl-glycyl-L-phenylalanyl-L-α-aminopentansulfonsäure [erfindungsgemäss] | 327,7 | 7,8 | 42,0 |
| L-Tyrosyl-D-alanyl-glycyl-L-phenylalanyl-DL-α-aminopentansulfonsäure [erfindungsgemäss] | 679,6 | 5,6 | 121,3 |
| L-Tyrosyl-D-alanyl-glycyl-L-phenylalanyl-L-phenylalanyl-L-norleucin* [Vergleichssubstanz] {deutsche Offenlegungsschrift 27 40 699, Beispiel 1} | 183,5 | 2,8 | 65,5 |
| L-Tyrosyl-D-methionyl-glycyl-L-phenylalanyl-L-α-aminopentanphosphonsäure [erfindungsgemäss] | 188,2 | 1,5 | 125,5 |
| L-Tyrosyl-D-methionyl-glycyl-L-phenylalanyl-L-α-aminopentansulfonsäure [erfindungsgemäss] | 374,5 | 9,9 | 37,8 |
| L-Tyrosyl-D-methionyl-glycyl-L-phenylalanyl-L-norleucin* [Vergleichssubstanz] | 305,8 | 4,8 | 63,7 |
| L-Tyrosyl-D-norleucyl-glycyl-L-phenylalanyl-L-α-aminopentanphosphonsäure [erfindungsgemäss] | 774,3 | 4,9 | 158,0 |

Fortsetzung der Tabelle I

| Verbindung | Opiatwirksamkeit an Maussamenleiterpräparaten | Opiatwirksamkeit am Krummdarm von Meerschweinchen | Opiatwirksamkeit an Maussamenleiterpräparaten / Opiatwirksamkeit am Krummdarm von Meerschweinchen |
|---|---|---|---|
| L-Tyrosyl-D-norleucyl-glycyl-L-phenylalanyl-L-α-aminopentansulfonsäure [erfindungsgemäss] | 873,8 | 21,6 | 40,45 |
| L-Tyrosyl-D-norleucyl-glycyl-L-phenylalanyl-L-norleucin* [Vergleichssubstanz] | 141,15 | 3,1 | 45,5 |

* S. Bajusz und Mitarbeiter: Acta Biochem. Biophys. Acad. Sci. Hung. 11 [1976], 305

Die erfindungsgemässen Verbindungen sind also stabile, gegenüber der Carboxypeptidase resistende Encephalin-Analoge, deren Optiatwirkung von einem anderen Charakter als die der Verbindungen der deutschen Offenlegungsschrift 2 740 699 (eher mit einer Wirkung von der Art des natürlichen β-Endorphines) ist. Hinzukommt noch, dass die erfindungsgemässen Verbindungen im Gegensatz zu denen der genannten Druckschrift mit ihrer beträchtlichen schmerzstillenden beziehungsweise analgetischen Wirkung keine solche Wirkung haben.

Ferner wurde festgestellt, dass die Salze der erfindungsgemässen Pentapeptide der allgemeinen Formel I mit bestimmten 2wertigen Metallen, zum Beispiel die Zink$^{++}$- und Cu$^{++}$-Salze von L-Tyrosyl-D-norleucyl-glycyl-L-phenylalanyl-L-α-aminopentanphosphonsäure und L-Tyrosyl-D-norleucyl-glycyl-L-phenylalanyl-L-α-aminopentansulfonsäure, einen besonders grossen Quotienten von

$$\frac{\text{Opiatwirksamkeit an Maussamenleiterpräparaten}}{\text{Opiatwirksamkeit am Krummdarm von Meerschweinchen}}$$

aufweisen. Die Opiatwirksamkeiten dieser Salze an Maussamenleiterpräparaten ist um 1 Grössenordnung höher als die der in Form von Zwitterionen vorliegenden Pentapeptide oder ihrer in Pufferlösungen entstehenden Natrium-, Kalium-, Magnesium- oder Calciumsalze, während die am Krummdarm von Meerschweinchen messbare Wirksamkeit durch die Bindung der Cu$^{++}$- beziehungsweise Zn$^{++}$-Ionen nicht beeinflusst wird.

Die erfindungsgemässen Verbindungen verändern den Brenzcatechinamingehalt des Kernes von Amygdala centralis in selektiver Weise. Da diese Kerngruppe einen hohen Encephalingehalt und eine grosse Opiatrezeptordichte hat und nicht Teil des das Schmerzgefühl spezifisch beeinflussenden Systemes ist, können die erfindungsgemässen Verbindungen folgende Funktionen spezifisch beeinflussen: Nahrungsaufnahme, Emotionalität beziehungsweise Erregbarkeit der Gefühle, soziales Verhalten, Lern- und Gedächtnisprozesse sowie endokrine und vegetative Regelungssysteme.

Ferner wurde für die erfindungsgemässen Verbindungen L-Tyrosyl-D-alanyl-glycyl-L-phenylalanyl-L-α-aminopentanphosphonsäure und L-Tyrosyl-D-norleucyl-glycyl-L-phenylalanyl-D-α-aminopentansulfonsäure an mit 5-Äthyl-5-(1′-methyl-n-butyl)-barvitursäure [Pentobarbital] (35 mg/kg intraperitoneal) narkotisierten Katzen (Gewicht: 3 bis 4 kg) überraschenderweise eine zentrale blutdrucksenkende Wirkung nachgewiesen. Ausser den Blutdruck- und Pulsänderungen wurde auch der durch Okklusion der Carotis ausgelöste Vasomotor-Reflex untersucht. Die erfindungsgemässen Pentapeptide führten in der angewandten Dosis eine von geringfügiger Bradykardie begleitete Senkung des Blutdruckes herbei. Der durch Carotisokklusion ausgelöste Vasomotor-Reflex wurde von beiden Verbindungen gehemmt. Das Maximum der Blutdruck- und Pulsänderung trat 5 bis 10 Minuten nach der Verabreichung ein, die Reflexhemmung war in der 20sten Minute maximal. Die Blutdruck- und Pulsänderung dauerte 20 bis 25 Minuten, die Reflexhemmung 40 bis 60 Minuten. Da die genannten beiden erfindungsgemässen Verbindungen an Ratten auch in höheren Dosen (3 bis 30 mg/kg intravenös) nicht schmerzlindernd beziehungsweise analgetisch wirken, ist die blutdrucksenkende Wirkung als selektiv anzusehen. Um die mit 0,1 mg/kg der genannten erfindungsgemässen Verbindungen erreichbare blutdrucksenkende Wirkung mit Morphin auszulösen, muss das Morphin in einer Dosis von mehr als 1 mg/kg verwendet werden. Die Ergebnisse die auf den Kreislauf ausgeübten Wirkungen der genannten beiden erfindungsgemässen Verbindungen und der Vergleichssubstanz Morphin betreffen, sind in der folgenden Tabelle II zusammengestellt.

Tabelle II

| Verbindung | Intravenöse Dosis in mg/kg | Blutdrucksenkung in mm Hg | Verminderung des Pulses in Schlägen/Minute | Durch Carotisokklusion ausgelöste Vasomotor-Reflex-Hemmung |
|---|---|---|---|---|
| L-Tyrosyl-D-norleucyl-glycyl-L-phenylalanyl-D-α-aminopentansulfonsäure [erfindungsgemäss] | 0,1 | 32,5±1,4 | 25,0±2,0 | über 50% |
| L-Tyrosyl-D-alanyl-glycyl-L-phenylalanyl-L-α-aminopentanphosphonsäure [erfindungsgemäss] | 0,1 | 22,5±1,4 | 21,3±4,3 | über 50% |
| Morphin | 0,2 | 6,7±2,1 | – | – |
| [Vergleichs- | 1,0 | 16,7±3,2 | 10,2±2,3 | unter 50% |
| substanz] | 5,0 | 33,3±6,7 | 16,7±3,5 | über 50% |

Weiterhin wurde überraschenderweise festgestellt, dass zum Beispiel die erfindungsgemässe Verbindung L-Tyrosyl-D-alanyl-glycyl-L-phenylalanyl-L-α-aminopentanphosphonsäure in einer intravenösen Dosis von 0,2 bis 1,0 mg/kg bei Ratten die Prolactinsekretion auf das 2 bis 5fache erhöht, was darauf hinweist, dass die erfindungsgemässen Verbindungen die endokrine Regulierung zu beeinflussen vermögen.

Schliesslich wurde überraschenderweise festgestellt, dass die erfindungsgemässen Verbindungen von Carboxypeptidasen nicht zersetzt (hyrolysiert) werden können. Dies bedeutet, dass durch den Austausch der Aminocarbonsäureeinheit gegen eine Aminophosphon- beziehungsweise Aminosulfonsäureeinheit oder anders ausgedrückt durch den Austausch der

endständigen Carboxylgruppe 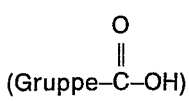 gegen eine

Phosphonsäuregruppe 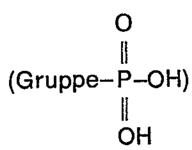

beziehungsweise

Sulfonsäuregruppe 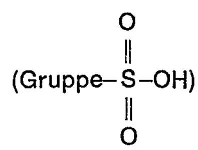

Pentapeptide, welche carboxypeptidase-resistent sind und trotzdem eine endständige Säuregruppe aufweisen, vorliegen. Früher konnte die Enzymresistenz nur dadurch erreicht werden, dass der endständigen Aminosäure zum Beispiel durch Amidieren, Verestern oder Reduktion zum Carbinol ihr Säurecharakter entzogen wurde. Derartige Abwandlungen können aber wie es sich bei der Untersuchung der Pentapeptide vom Encephalintyp ergab, das ursprüngliche Wirkungsspektrum, den biologischen Charakter der Pentapeptide bedeutend verändern.

Auch haben die erfindungsgemässen Verbindungen eine äusserst geringe Toxizität. So wurde bei subkutaner Verabreichung von L-Tyrosyl-D-alanyl-glycyl-L-phenylalanyl-L-α-aminopentansulfonsäure und L-Tyrosyl-D-norleucyl-glycyl-L-phenylalanyl-L-α-aminopentansulfonsäure an Mäuse nicht einmal in einer Dosis von 100 mg/kg irgendeine toxische Wirkung festgestellt.

Die Erfindung wird an Hand der folgenden Beispiele näher erläutert. Die in diesen angegebenen $R_f$-Werte wurden durch Schichtchromatographie an Silicagel (Kieselgel G, Reanal, Budapest) mit den folgenden Lösungsmittelgemischen beziehungsweise dem folgenden Lösungsmittel bestimmt:

| Lösungsmittelgemisch beziehungsweise Lösungsmittel | Volumverhältnis der Lösungsmittel der Lösungsmittelgemische |
|---|---|
| 1. Äthylacetat/Pyridin/Essigsäure/Wasser | 960 : 20 : 6 : 11 |
| 2. Äthylacetat/Pyridin/Essigsäure/Wasser | 240 : 20 : 6 : 11 |
| 3. Äthylacetat/Pyridin/Essigsäure/Wasser | 120 : 20 : 6 : 11 |
| 4. Äthylacetat/Pyridin/Essigsäure/Wasser | 80 : 20 : 6 : 11 |
| 5. Äthylacetat/Pyridin/Essigsäure/Wasser | 60 : 20 : 6 : 11 |
| 6. Äthylacetat/Pyridin/Essigsäure/Wasser | 30 : 20 : 6 : 11 |

| Lösungsmittelgemisch beziehungsweise Lösungsmittel | Volumverhältnis der Lösungsmittel der Lösungsmittelgemische |
|---|---|
| 7. Äthylacetat/Pyridin/Essigsäure/Wasser | 60 : 20 : 6 : 5,5 |
| 8. Äthylacetat/Pyridin/Essigsäure/Wasser | 30 : 20 : 6 : 5,5 |
| 9. n-Butanol Essigsäure/Wasser | 4 : 1 : 1 |
| 10. Chloroform/Methanol/Essigsäure | 90 : 5 : 5 |
| 11. Chloroform/Methanol/Essigsäure | 3 : 1 : 1 |
| 12. Chloroform/n-Hexan/Essigsäure | 8 : 1 : 1 |
| 13. Chloroform/Aceton | 9 : 1 |
| 14. Chloroform | |

Beispiel 1

L-Tyrosyl-glycyl-glycyl-L-phenylalanyl-L-α-aminopentanphosphonsäure

Schritt 1: L-Phenylalanyl-D-α-aminopentanphosphonsäure

39,8 g (150 mMol) tert.-Butyloxycarbonyl-L-phenylalanin und 16,7 ml (150 mMol) N-Methylmorpholin werden in einem Gemisch aus 100 ml Dioxan und 100 ml Tetrahydrofuran gelöst. Die Lösung wird auf −10 °C gekühlt und unter Rühren mit 19,2 g (145 mMol) Chlorkohlensäure-isobutylester, 10 Minuten später mit der Lösung von 16,7 g (100 mMol) von DL-α-Aminopentanphosphonsäure in 55 ml 4n Natronlauge versetzt. Das Reaktionsgemisch wird bei Temperaturen zwischen −5 und −10 °C 5 Stunden lang gerührt und dann unter vermindertem Druck auf etwa 70 ml eingeengt. Der Rückstand wird in einem Zweiphasensystem aus 500 ml 0,5n Schwefelsäure und 500 ml Äthylacetat gelöst. Die wässrige Phase wir mit 2 × 100 ml Äthylacetat ausgeschüttelt, dann werden die organischen Phasen vereinigt und fünfmal mit je 100 ml 20%igem wässrigem Pyridin extrahiert. Aus der Äthylacetatlösung wird nach Waschen mit 0,5n Schwefelsäure, Trocknen über wasserfreiem Natriumsulfat und Eindampfen nicht umgesetztes tert.-Butyloxycarbonyl-L-phenylalanin (16 g; 60 mMol) zurückgewonnen. Die wässrigen Pyridinextrakte werden vereinigt und unter vermindertem Druck eingedampft. Der Rückstand wird in 200 ml Äthylacetat und so viel 0,5n Schwefelsäure gelöst, dass der pH-Wert der wässrigen Phase 2 beträgt. Die wässrige Phase wird mit 2 × 50 ml Äthylacetat ausgeschüttelt, die Lösungen werden vereinigt, mit 50 ml Wasser gewaschen und unter vermindertem Druck eingedampft. Von dem Rückstand wird auch ein Gemisch von 10 ml Äthanol und 40 ml Benzol abdestilliert, dann wird der Rückstand in 50 ml Trifluoressigsäure gelöst. Die Lösung wird 30 Minuten stehen gelassen und dann eingedampft. Der Rückstand wird mit Äther verrieben, abfiltriert und im Vakuumexsikkator über Kaliumhydroxyd getrocknet. Das Produkt wird bei 40–50 °C in 70 ml Essigsäure gelöst, die Lösung mit 125 ml 40–50 °C warmem Wasser verdünnt und bei Raumtemperatur über Nacht stehen gelassen. Die ausgeschiedenen Kristalle werden abfiltriert und mit 3 × 10 ml 30%iger wässriger Essigsäure gewaschen. Die essigsaure Mutterlauge und die Waschflüssigkeit werden aufbewahrt (für Beispiel 1, Schritt 2).

Das kristalline Produkt wird im Vakkumexsikkator über Kaliumhydroxyd getrocknet. 11,0 g (35 mMol, 70%) L-Phenylalanyl-D-α-aminopentanphosphonsäure werden erhalten, die bei 262 °C schmilzt. $[a]_D^{20} = +68{,}9°$ (c = 1, n Natronlauge), $R_f^6$: 0,45–0,50.

Schritt 2: L-Phenylalanyl-L-α-aminopentanphosphonsäure

Die in Schritt 1 erhaltene essigsaure Mutterlauge wird mit der essigsauren Waschflüssigkeit vereinigt und unter vermindertem Druck eingedampft. Von dem kristallinöligen Rückstand werden zweimal je 50 ml Dioxan abdestilliert. Der Rückstand wird in 50 ml Dioxan suspendiert, abfiltriert, zuerst mit Dioxan, dann mit Äther gewaschen und im Vakuumexsikkator über Kaliumhydroxy getrocknet. 10,9 g (34,8 mMol, 69,6%) Produkt werden erhalten, das bei 251–253 °C schmilzt. $[\alpha]_D^{20} = 53{,}1°$ (c = 1, n Natronlauge); $R_f^6$: 0,48–0,53.

Schritt 3: L-Tyrosyl-glycyl-glycyl-phenylalanyl-L-α-aminopentanphosphonsäure

0,95 g (2,4 mMol) des geschützten Tripeptides tert.-Butyloxycarbonyl-L-tyrosyl-glycyl-glycyn und 0,27 mMol (2,4 mMol) N-Methyl-norpholin werden in 5 ml Dimethylformamid gelöst. Zu der Lösung werden bei −10 °C unter Rühren zuerst 0,31 ml (2,4 mMol) Chlorkohlensäureisobutylester und nach 10 Minuten die Lösung von 0,63 g (2 mMol) des freien Dipeptides (d. h. der gemäss Schritt 2 erhaltenen L-Phenylalanyl-L-α-aminopentanphosphonsäure) und 0,56 ml (4 mMol) Triäthylamin in einem Gemisch aus 4 ml Dimethylformamid und 0,5 ml Wasser zugegeben.

Das Reaktionsgemisch wird bei Temperaturen zwischen −5 und −10 °C 3–4 Stunden lang gerührt und dann unter vermindertem Druck eingedampft. Der Rückstand wird in 20 ml 5%igem wässrigem Pyridin gelöst und die Lösung mit 0,3 g (2,3 mMol) Calciumchlorid-hydrat in 5 ml Wasser versetzt. Der entstandene Niederschlag wird abfiltriert, mit 5 × 5 ml Wasser gewaschen und dann mit 10 ml n Schwefelsäure und 30 ml Äthylacetat versetzt. Das System wird intensiv gerührt, das ausgeschiedene Calciumsulfat wird abgetrennt, und die Phasen werden voneinander getrennt. Die wässrige Phase wird mit 2 × 10 ml Äthylacetat ge-

waschen, die Waschflüssigkeit wird mit der abgetrennten organischen Phase vereinigt und unter vermindertem Druck eingedampft. Der Rückstand wird in 10 ml Trifluoressigsäure gelöst und die Lösung 30 Minuten lang stehengelassen. Dann wird die Lösung unter vermindertem Druck eingedampft, und von dem Rückstand werden 2 × 10 ml Essigsäure abdestilliert. Dann wird der Rückstand im Vakuumexsikkator über Kaliumhydroxyd getrocknet. Dann wird das Produkt mit 5 ml Wasser verrieben, abfiltriert, mit 3 × 3 ml Wasser gewaschen und im Vakuumexsikkator über Kaliumhydroxyd getrocknet. 0,85 g (72%) Produkt werden erhalten. Schmp.: 203–205 °C; $[\alpha]_D^{20} = -8°$ (c = 1, n Natronlauge); $R_f^5$: 0,30–0,35

Die als Ausgangsstoff eingesetzte DL-α-Aminopentanphosphonsäure ist wie folgt beschrieben erhalten worden.

45 g (300 mMol) Benzylurethan werden unter Rühren in einem Gemisch aus 60 ml Eisessig und 26,4 ml (300 mMol) Phosphortrichlorid gelöst. Die Lösung wird mit Eiswasser gekühlt und bei einer Temperatur unter 10 °C mit 52 ml (450 mMol) n-Valeraldehyd versetzt. Das Reaktionsgemisch wird evakuiert (3–4 × $10^4$ Pa). Die Temperatur der gekühlten Lösung sinkt zuerst auf 2–3 °C ab und steigt dann unter Entwicklung von Salzsäure auf 30–40 °C an. Die Kühlung wird entfernt, und das Reaktionsgemisch wird zwei Stunden lang auf 65–75 °C gehalten. Dabei wird das Gemisch immer zäher und erstarrt schliesslich. Nach 3–4 Stunden wird das Gemisch mit 18 ml Wasser versetzt und dann über Nacht bei Raumtemperatur und atmosphärischem Druck stehengelassen. Aus dem Zweiphasengemisch wird anschliessend die Essigsäure unter vermindertem Druck abdestilliert. Der Rückstand wird in einem Gemisch aus 500 ml Äthylacetat und 300 ml Wasser gelöst. Die organische Phase wird mit 2 × 100 ml Wasser, dann mit 2 × 100 ml n Salzsäure und schliesslich mit 3 × 150 ml n Natronlauge ausgeschüttelt. Die Waschlaugen werden vereinigt, mit 50 ml Äthylacetat gewaschen, mit 5n Schwefelsäure auf pH 1 angesäuert und dann mit 3 × 200 ml Äthylacetat ausgeschüttelt. Die organischen Phasen werden vereinigt, über wasserfreiem Natriumsulfat getrocknet und unter vermindertem Druck eingedampft. Der Rückstand wird in 500 ml Diisopropyläther gelöst und die Lösung mit 60 ml Dicyclohexylamin versetzt. Die ausgeschiedenen Kristalle werden abfiltriert, mit Diisopropyläther gewaschen und an der Luft getrocknet. Dann wird das Produkt in 150 ml kochendem Äthanol gelöst, die Lösung wird auf 40–50 °C gekühlt und mit 800 ml Diisopropyläther versetzt. Die ausgeschiedenen Kristalle werden abfiltriert, mit Diisopropyläther gewaschen und an der Luft getrocknet. Das erhaltene Salz (97 g, Schmp.: 147–149 °C) wird in 50 ml Äthylacetat und 300 ml 5n Schwefelsäure gelöst, die organische Phase wird zweimal mit 300 ml 5n Schwefelsäure gewaschen, dann über wasserfreiem Natriumsulfat getrocknet und unter vermindertem Druck eingedampft. Der Rückstand wird aus Petroläther kristallisiert. 54,3 g (60%) Benzyloxycarbonyl-DL-α-aminopentanphosphonsäure werden erhalten, das bei 98–100 °C schmilzt. $R_f^5$: 0,45–0,55.

45,3 g (150 mMol) der wie vorstehend beschrieben hergestellten Benzyloxycarbonyl-DL-α-aminopentanphosphonsäure werden in 500 ml Wasser suspendiert. Zu der Suspension werden 42 ml (300 mMol) Triäthylamin gegeben, und das Gemisch wird in Gegenwart von Palladiumaktivkohle als Katalysator hydriert. Nach Ablauf der Reaktion wird der Katalysator abfiltriert, mit Wasser gewaschen, das Waschwasser mit dem Filtrat vereinigt und dieses unter vermindertem Druck eingedampft. Der Rückstand wird in 150 ml siedender wässriger n Essigsäure gelöst, mit Aktivkohle geklärt und nach Zusatz von 900 ml Äthanol bei 0 °C stehengelassen. Die ausgeschiedenen Kristalle werden abfiltriert, mit Äthanol gewaschen und im Vakuumexsikkator getrocknet. 23,1 g (92%) DL-α-Aminopentanphosphonsäure werden erhalten. Schmp.: 275–278 °C; $R_f^5$: 0,0–0,1; $R_f^6$: 0,23–0,33.

Das als Ausgangsstoff verwendete geschützte Tripeptid tert.-Butyloxycarbonyl-L-tyrosyl-glycyl-glycin ist wie folgt beschrieben erhalten worden.

14,2 g (42 mMol) Glycin-benzylester-p-toluolsulfonat werden in 100 ml Dimethylformamid gelöst, zu der Lösung werden 4,6 ml (42 mMol) N-Methylmorpholin und 14,2 g (40 mMol) tert.-Butyloxycarbonylglycin-2,4,5-trichlorphenylester gegeben, und das Reaktionsgemisch wird über Nacht bei Raumtemperatur gerührt. Dann wird das Gemisch eingedampft, der Rückstand wird in einem Zweiphasengemisch aus 150 ml Äthylacetat und 50 ml Wasser gelöst. Die organische Phase wird mit 2 × 30 ml eiswassergekühlter n Salzsäure, danach mit Wasser gewaschen, über Natriumsulfat getrocknet und schliesslich eingedampft. Der Rückstand wird mit einem im Volumverhältnis 1 : 1 bereiteten Gemisch aus Diisopropyläther und Petroläther verrieben, abfiltriert, mit dem gleichen Lösungsmittelgemisch gewaschen und im Exsikkator getrocknet. 11,0 g (82,2%) tert.-Butyloxycarbonyl-glycyl-glycin-benzylester werden erhalten. Schmp.: 83 °C; $R_f^{14}$: 0,1–0,2.

10,15 g (31,5 mMol) des wie vorstehend beschriebenen erhaltenen geschützten Dipeptidesters tert.-Butyloxycarbonyl-glycyl-glycin-benzylester werden in 100 ml 11–15% Chlorwasserstoff enthaltendem Äthylacetat gelöst. Die Lösung wird eine halbe Stunde stehengelassen und dann unter vermindertem Druck eingedampft. Der Rückstand wird im Vakuumexsikkator über Kaliumhydroxyd getrocknet. Das erhaltene Produkt (7,8 g; $R_f^3$: 0,1–0,2) wird in 35 ml Dimethylformamid gelöst, die Lösung wird mit 3,4 ml (30,13 mMol) N-Methylmorpholin und 15,1 g (30 mMol) tert.-Butyloxycarbonyl-L-tyrosin-pentachlorphenylester versetzt und 16–20 Stunden lang stehen gelassen. Innerhalb der ersten fünf Stunden werden dem Reaktionsgemisch in fünf Portionen 3,3 ml (30 mMol) N-Methylmorpholin zugesetzt. Die Lösung wird unter vermindertem Druck eingedampft, der Rückstand in einem Zweiphasensystem von 250 ml Äthylacetat und 50 ml Wasser gelöst, die organische Phase wird zuerst mit 2 × 50 ml 5%iger Na-

triumhydrogencarbonatlösung, dann mit 2 × 50 ml Wasser, mit 2 × 50 ml eiswassergekühlter n Salzsäure und schliesslich erneut mit Wasser gewaschen, über Natriumsulfat getrocknet, mit Aktivkohle geklärt und schliesslich unter vermindertem Druck eingedampft. Der Eindampfrückstand wird mit Diisopropyläther verrieben, abfiltriert, mit Diisopropyläther gewaschen und im Vakuumexsikkator getrocknet. Der auf diese Weise erhaltene geschützte Tripeptidester ($R_f^{10}$: 0,65–0,75) wird in 150 ml Methanol gelöst und in Gegenwart von Palladiumaktivkohle hydriert. Danach wird der Katalysator durch Filtrieren entfernt, die methanolische Lösung unter vermindertem Druck eingedampft und der Rückstand mit einem im Volumverhältnis 1 : 1 bereiteten Gemisch aus Diisopropyläther und Diäthyläther verrieben, abfiltriert und im Vakuumexsikkator getrocknet. 8,35 g (70,3%) tert.-Butyloxycarbonyl-L-tyrosyl-glycyl-glycin werden erhalten. Schmp.: 92–94 °C; $R_f^9$: 0,2–0,3.

Beispiel 2

L-Tyroxyl-glycyl-glycyl-L-phenylalanyl-L-α-aminopentansulfonsäure

Schritt 1: Benzyloxycarbonyl-L-phenylalanyl-DL-α-aminopentansulfonsäure

101,8 g (340 mMol) Benzyloxycarbonyl-L-phenylalanin werden in 950 ml Dimethylformamid gelöst. Die Lösung wird auf −10 °C gekühlt und bei dieser Temperatur unter Rühren mit 37,8 ml (340 mMol) N-Methylmorpholin und 41,5 ml (340 mMol) Pivaloylchlorid versetzt. Das Gemisch wird 10 Minuten lang gerührt und dann auf −30 °C gekühlt. Bei dieser Temperatur werden 51,7 g (309 mMol) DL-α-Aminopentansulfonsäure und 43,3 ml (309 mMol) Triäthylamin zugegeben. Das Gemisch wird bei −10 °C 10 Minuten lang, bei 0 °C eine Stunde und dann bei Raumtemperatur über Nacht weiter gerührt. Dann wird das Reaktionsgemisch unter vermindertem Druck eingedampft und der Rückstand in 1500 ml Wasser gelöst. Die Lösung wird mit 3 × 300 ml Diäthyläther gewaschen, unter Kühlung mit Eiswasser mit konzentrierter wässriger Salzsäure auf pH 2 angesäuert und mit 3 × 300 ml wassergesättigtem n-Butanol ausgeschüttelt. Die vereinigten butanolischen Lösungen werden mit 50 ml Wasser, das mit n-Butanol gesättigt ist, gewaschen, dann mit Aktivkohle geklärt und unter vermindertem Druck eingedampft. 82,6 g (85%) Produkt werden erhalten. $R_f^3$: 0,2–0,3.

Schritt 2: L-Phenylalanyl-D-α-aminopentansulfonsäure

Die gesamte Menge des gemäss Schritt 1 erhaltenen geschützten Dipeptides (82,6 g, 262,7 mMol) wird in 3000 ml Methanol gelöst und in Gegenwart von Palladiumaktivkohle hydriert. Am Ende der Reaktion (die in der Lösung nachweisbare Substanz hat einen $R_f^2$-Wert von 0,05 bis 0,15) werden der Katalysator und die ausgefallene feste Substanz abfiltriert und mit 4 × 300 ml Methanol gewaschen. Die methanolischen Lösungen werden vereinigt und aufbewahrt (für Schritt 3). Das abfiltrierte, mit Katalysator verunreinigte Produkt wird in 300 ml 50%igem Dimethylformamid suspendiert, die Suspension auf 70–80 °C erwärmt und warm filtriert. Der abfiltrierte Katalysator wird mit 2 × 50 ml warmem 50%igem Dimethylformamid gewaschen. Die vereinigten wässrigen Dimethylformamidphasen werden unter vermindertem Druck eingedampft. Der kristalline Rückstand wird in Methanol suspendiert, abfiltriert, mit Methanol und Diäthyläther gewaschen und im Vakuumexsikkator getrocknet. 34,5 g (83%) L-Phenylalanyl-D-α-aminopentansulfonsäure werden erhalten. Schmp.: 284–285°; $[\alpha]_D^{20} = 127,5°$ (c = 1, n Natronlauge).

Schritt 3: L-Phenylalanyl-L-α-aminopentansulfonsäure

Die im Schritt 2 erhaltene methanolische Lösung wird unter vermindertem Druck eingedampft. Der kristalline Rückstand wird in 100 ml Aceton suspendiert, mit Aceton gewaschen und im Vakuumexsikkator getrocknet. Das erhaltene Produkt (34,6 g) wird in 700 ml 80%igem wässrigem Äthanol suspendiert, die Suspension wird bis zum Siedepunkt erhitzt, dann vom Unlöslichen abfiltriert und das Filtrat über Nacht im Eisschrank stehengelassen. Die Kristalle werden abfiltriert, mit kaltem 80%igem wässrigem Äthanol gewaschen und im Vakuumexsikkator getrocknet. Das Umristallisieren wird noch zweimal (mit 600 bzw. 500 ml des gleichen Lösungsmittels) wiederholt. 20,7 g (50%) L-Phenylalanyl-L-α-aminopentansulfonsäure werden erhalten, die bei 220 °C schmilzt. $[\alpha]_D^{20} = -73,1°$ (c = 1, n Natronlauge); $R_f^{11}$: 0,4–0,5.

Schritt 4: tert.- Butyloxycarbonyl-L-tyrosyl-glycyl-glycyl-L-phenylalanyl-L-α-aminopentansulfonsäure-N-methylmorpholinsalz

0,95 g (3 mMol) des gemäss Schritt 3 erhaltenen Dipeptides werden in 10 ml Dimethylformamid suspendiert. Zu der Suspension werden 0,35 ml (3 mMol) N-Methylmorpholin und 2,0 g (3,15 mMol) tert.-Butyloxycarbonyl-L-tyrosyl-glycyl-glycin-pentachlorphenylester zugegeben. Das Reaktionsgemisch wir 16–20 Stunden lang gerührt, wobei innerhalb der ersten zwei Stunden in fünf Portionen 0,35 ml (3,15 mMol) N-Methylmorpholin zugesetzt werden. Das Reaktionsgemisch wird unter vermindertem Druck eingedampft und der Rückstand in einem Zweiphasensystem aus 20 ml Äthylacetat und 60 ml Wasser gelöst. Die wässrige Phase wird mit 2 × 20 ml Äthylacetat gewaschen, mit Aktivkohle geklärt und unter vermindertem Druck eingedampft. Der Eindampfrückstand wird in 20 ml Äthanol gelöst und die Lösung erneut eingedampft. Der Rückstand wird unter Kühlung mit Diäthyläther verrieben, abfiltriert, mit Diäthyläther gewaschen und im Vakuumexsikkator getrocknet. 1,82 g (76,5%) Produkt werden erhalten, das bei 130 °C schmilzt. $R_f^7$: 0,25–0,35; $[\alpha]_D^{20} = -24,8°$ (c = 1, Dimethylformamid).

Schritt 5: L-Tyrosyl-glycyl-glycyl-L-phenylalanyl-L-α-aminopentansulfonsäure

1,4 g (1,75 mMol) des gemäss Schritt 4 erhaltenen Salzes des geschützten Pentapeptides werden in 10 ml Trifluoressigsäure gelöst und die Lösung wird bei Raumtemperatur eine halbe Stunde lang stehengelassen. Dann wird die Lösung unter vermindertem Druck eingedampft. Der Rückstand wird mit Diäthyläther verrieben, abfiltriert, mit Diäthyläther, dann mit Äthylacetat, danach erneut mit Diäthyläther gewaschen und im Vakuumexsikkator getrocknet. 1,05 g (99%) Produkt werden erhalten. Schmp.: 192–194 °C. $R_f^5$: 0,20–0,35; $R_f^8$: 0,35–0,45; $[\alpha]_D^{20} = +6,01°$ (c = 1, 90%ige Essigsäure).

Die als Ausgangsstoff eingesetzte DL-Aminopentansulfonsäure ist wie folgt beschrieben erhalten worden.

52 g (500 mMol) Natriumbisulfit werden in 250 ml warmem Wasser gelöst, die Lösung wird mit 53,6 ml (510 mMol) n-Valeraldehyd versetzt und das entstehende orangefarbene Reaktionsgemisch auf dem Dampfbad so lange gerührt, bis es sich entfärbt. Dann wird die Lösung auf Raumtemperatur abgekühlt, mit 400 ml konzentriertem wässrigem Ammoniak versetzt und eine Stunde lang bei Raumtemperatur, danach über Nacht im Eisschrank stehen gelassen. Dann wird das Reaktionsgemisch mit 100 ml Diäthyläther ausgeschüttelt, mit Aktivkohle geklärt und unter Kühlen mit konzentrierter wässriger Salzsäure auf pH 3 angesäuert. Die ausgeschiedene kristalline Substanz wird abfiltriert, zuerst mit Wasser, dann mit Diäthyläther gewaschen und im Vakuumexsikkator getrocknet. 47,6 g (56,8%) DL-α-Aminopentansulfonsäure werden erhalten. Schmp.: 142 °C; $R_f^9$: 0,3–0,4.

Der als Ausgangsstoff eingesetzte tert.-Butyloxycarbonyl-L-tyrosyl-glycyl-glycin-pentachlorphenylester ist wie folgt beschrieben erhalten worden.

6,0 g (15 mMol) des wie im Beispiel 1 bei der Herstellung von dessen entsprechendem Ausgangsstoff beschrieben hergestellten geschützten Tripeptides tert.-Butyloxycarbonyl-L-tyrosyl-glycyl-glycin und 4,3 g (16 mMol) Pentachlorphenol werden in 50 ml Dimethylformamid gelöst. Die Lösung wird mit Eiswasser auf etwa 5 °C gekühlt, mit 3,4 g (16,5 mMol) Dicyclohexylcarbodiimid versetzt und eine Stunde lang unter Kühlung, dann über Nacht bei Raumtemperatur gerührt. Der ausgeschiedene Dicyclohexylharnstoff wird abfiltriert, mit Dimethylformamid gewaschen, die Waschflüssigkeit mit dem Filtrat vereinigt und dieses eingedampft. Der Eindampfrückstand wird in 100 ml Äthanol suspendiert, die Suspension eine halbe Stunde lang auf dem Dampfbad erwärmt, nach Kühlen mit Eiswasser filtriert, das Produkt mit gekühltem Äthanol gewaschen und im Vakuumexsikkator getrocknet. 6,7 g (69%) Produkt werden erhalten. Schmp.: 186 °C; $R_f^{10}$: 0,6–0,7.

Beispiel 3

L-Tyrosyl-D-alanyl-glycyl-L-phenylalanyl-L-α-aminopentanphosphonsäure

Ausgehend von 0,98 g (2,4 mMol) tert.-Butyloxycarbonyl-L-tyrosyl-D-alanyl-glycin (Pless u.a.: Helv. Chim. Acta 62, 398 [1979]) und 0,63 g (2 mMol) L-Phenylalanyl-L-α-aminopentanphosphonsäure (Beispiel 1, Schritt 2 wird auf die im Schritt 3 des Beispiels 1 beschriebene Weise das obige Produkt in 0,85 g (70%) Ausbeute erhalten. Schmp.: 255–257 °C; $[\alpha]_D^{20} = +24,5°$ (c = 1, n Natronlauge); $R_f^5$: 0,30–0,40.

Beispiel 4

L-Tyrosyl-D-alanyl-glycyl-L-phenylalanyl-L-α-aminopentansulfonsäure

Schritt 1: tert.-Butyloxycarbonyl-L-tyrosyl-D-alanyl-glycyl-L-phenylalanyl-L-α-aminopentansulfonsäure-triäthylaminsalz

0,95 g (3 mMol) des gemäss Beispiel 2, Schritt 3, erhaltenen Dipeptides und 2,1 g (3,15 mMol) tert.-Butyloxycarbonyl-L-tyrosyl-D-alanyl-glycin-pentachlorphenylester werden auf die im Schritt des Beispieles 2 beschriebene Weise kondensiert. Als tertiäre Base werden 0,4 beziehungsweise 0,45 ml Triäthylamin verwendet. 1,99 g (82,2%) des genannten Produktes werden erhalten. Schmp.: 125 °C; $R_f^5$: 0,35–0,45; $[\alpha]_D^{20} = -25,1°$ (c = 1, Dimethylformamid).

Schritt 2: L-Tyrosyl-D-alanyl-glycyl-L-phenylalanyl-L-α-aminopentansulfonsäure

1,4 g (1,75 mMol) des gemäss Schritt 1 erhaltenen Salzes des geschützten Pentapeptides werden auf die im Schritt 5 des Beispiels 2 beschriebene Weise umgesetzt mit dem Unterschied, dass der Niederschlag auch mit 10 ml Wasser gewaschen wird. 0,75 g (70,4%) Produkt werden erhalten. Schmp.: 235–240 °C; $R_f^5$: 0,35–0,45; $R_f^7$: 0,1–0,2; $[\alpha]_D^{29} = +5,9°$ (c = 1, 80%ige Essigsäure).

Der als Ausgangsstoff verwendete tert.-Butyloxycarbonyl-L-tyrosyl-D-alanyl-glycin-pentachlorphenylester ist wie folgt beschrieben erhalten worden.

6,15 g (15 mMol) tert.-Butyloxycarbonyl-L-tyrosyl-D-alanyl-glycin (Pless u.a.: Helv. Chim. Acta 62, 398 [1979]) werden auf die bei der Beschreibung der Herstellung des entsprechenden Ausgangsstoffes des Beispiels 2 angegebene Weise zum aktiven Ester umgesetzt. 7,6 g (76,6%) tert.-Butyloxycarbonyl-L-tyrosyl-D-alanyl-glycin-pentachlorphenylester werden erhalten. Schmp.: 222 °C; $R_f^{12}$: 0,1–0,2.

Beispiel 5

L-Tyrosyl-D-methionyl-glycyl-L-phenylalanyl-L-α-aminopentanphosphonsäure

Ausgehend von 1,13 g (2,4 mMol) tert.-Butyloxycarbonyl-L-tyrosyl-D-methionyl-glycin und 0,63 g (2 mMol) L-Phenylalanyl-L-α-aminopentanphosphonsäure (Beispiel 1, Schritt 2) werden auf die im Schritt 3 des Beispiels 1 beschriebene Weise 0,9 g (70%) Produkt erhalten. Schmp.: 193–195 °C; $[\alpha]_D^{20} = +15,6°$ (c = 1, n Natronlauge); $R_f^5$: 0,35–0,40.

Das als Ausgangsstoff verwendete tert.-Butyloxycarbonyl-L-tyrosyl-D-methionyl-glycin ist wie folgt beschrieben erhalten worden.

12,9 g (30 mMol) tert.-Butyloxycarbonyl-D-methionin-dicyclohexylaminsalz und 0,35 ml (3 mMol) N-Methylmorpholin werden in 120 ml Dimethylformamid gelöst. Die Lösung wird auf −10 °C gekühlt und bei dieser Temperatur unter Rühren mit 3,95 ml (30 mMol) Chlorkohlensäureisobutylester versetzt. Nach 10 Minuten wird das

Reaktionsgemisch auf −20 °C gekühlt, und die auf −20 °C gekühlte Lösung von 11,15 g (33 mMol) Glycinbenzylester-p-toluolsulfonat und 3,7 ml (33 mMol) N-Methylmorpholin in 50 ml Dimethylformamid wird zugegeben. Das Reaktionsgemisch wird bei −10 °C 10 Minuten, bei 0 °C eine Stunde lang und über Nacht bei Raumtemperatur gerührt. Dann wird das Gemisch unter vermindertem Druck eingeengt, in einem Zweiphasensystem aus 200 ml Äthylacetat und 100 ml 0,01n Schwefelsäure gelöst und die organische Phase mit 2 × 30 ml 0,01n Schwefelsäure und mit 2 × 30 ml Wasser gewaschen. Die Lösung wird über Natriumsulfat getrocknet, mit Aktivkohle geklärt und unter vermindertem Druck eingedampft. Der Eindampfrückstand wird mit Petroläther verrieben, abfiltriert, mit Petroläther gewaschen und im Vakuumexsikkator getrocknet. 11,3 g (94,8%) tert.-Butyloxycarbonyl-D-methionyl-glycyl-benzylester werden erhalten. Schmp.: 67–68 °C; $R_f^1$: 0,8–0,9; $R_f^{14}$: 0,35–0,45; $[\alpha]_D^{20} = +10{,}4°$ (c = 1, Dimethylformamid).

9,9 g (25 mMol) des wie vorstehend beschrieben erhaltenen geschützten Dipeptidesters werden in 50 ml 11–15% Chlorwasserstoff enthaltendem Äthylacetat gelöst. Nach 30 Minuten wird die Lösung unter vermindertem Druck eingedampft. Der Rückstand wird im Vakuumexsikkator über Kaliumhydroxyd getrocknet. Das erhaltene Produkt ($R_f^2$: 0,15–0,25) wird in 50 ml Dimethylformamid gelöst, und zu der Lösung werden 2,8 ml (25 mMol) N-Methylmorpholin und 14,55 g (27,5 mMol) tert.-Butyloxycarbonyl-L-tyrosin-pentachlorphenylester gegeben. Das Reaktionsgemisch wird über Nacht gerührt. Innerhalb der ersten beiden Stunden werden in 5 Portionen 3,05 g (27,5 mMol) N-Methylmorpholin zugesetzt. Dann wird das Reaktionsgemisch unter vermindertem Druck eingedampft, der Rückstand wird in einem Zweiphasengemisch aus 150 ml Äthylacetat und 50 ml Wasser gelöst. Die organische Phase wird mit 3 × 30 ml 5%iger Natriumhydrogencarbonatlösung, 3 × 30 ml Wasser, 3 × 30 ml eiswassergekühlter 0,1n Salzsäure und schliesslich wieder mit 3 × 30 ml Wasser gewaschen, dann über Natriumsulfat getrocknet, mit Aktivkohle geklärt und unter vermindertem Druck eingedampft. Der Eindampfrückstand ($R_f^{12}$: 0,1–0,2) wird in 100 ml Methanol gelöst und die Lösung mit 30 ml Wasser und 54 ml n Natronlauge versetzt. Man lässt das Reaktionsgemisch bei Raumtemperatur einen Tag stehen. Dann wird das Methanol unter vermindertem Druck abdestilliert, die wässrige Lösung wird mit 3 × 20 ml Äthylacetat ausgeschüttelt, mit fester Citronensäure auf pH 3 eingestellt, der ausgefallene Stoff abfiltriert, mit Wasser gewaschen und im Vakuumexsikkator getrocknet. 7,6 g (64,7%) tert.-Butyloxycarbonyl-L-tyrosyl-D-methionyl-glycin werden erhalten. Schmp.: 194 °C; $R_f^2$: 0,24–0,34; $[\alpha]_D^{20} = +11{,}44°$ (c = 1, Dimethylformamid).

Beispiel 6

L-Tyrosyl-D-methionyl-glycyl-L-phenylalanyl-L-α-aminopentansulfonsäure

Schritt 1: tert.-Butyloxycarbonyl-L-tyrosyl-D-methionyl-glycyl-L-phenylalanyl-L-α-aminopentansulfonsäure-N-methylmorpholinsalz

2,26 g (3,15 mMol) tert.-Butyloxycarbonyl-L-tyrosyl-D-methionyl-glycin-pentachlorphenylester und 0,95 g (3 mMol) Dipeptid (Beispiel 2, Schritt 3) werden auf die im Schritt 4 des Beispiels 2 beschriebene Weise kondensiert. 1,73 g (66,5%) Produkt werden erhalten. Schmp.: 124–127 °C; $R_f^5$: 0,35–0,45; $[\alpha]_D^{20} = -15°$ (c = 1, Dimethylformamid).

Schritt 2: L-Tyrosyl-D-methionyl-glycyl-L-phenylalanyl-L-α-aminopentansulfonsäure

1,5 g (1,75 mMol) des gemäss Schritt 1 erhaltenen Salzes werden auf die im Schritt 5 des Beispiels 2 beschriebene Weise weiter umgesetzt mit dem Unterschied, dass das Produkt auch noch mit 2 × 10 ml kochendem Wasser gewaschen wird. 0,78 g (66%) Produkt werden erhalten. Schmp.: 200–201 °C; $R_f^5$: 0,5–0,6; $R_f^7$: 0,2–0,3; $[\alpha]_D^{20} = -20°$ (c = 1, Trifluoressigsäure).

Der als Ausgangsstoff verwendete tert.-Butyloxycarbonyl-L-tyrosyl-D-methionyl-glycin-pentachlorphenylester ist wie folgt beschrieben erhalten worden.

Aus 7,05 g (15 mMol) tert.-Butyloxycarbonyl-L-tyrosyl-D-methionyl-glycin (Beispiel 5, Beschreibung der Herstellung des Ausgangsstoffes) wird auf die bei der Beschreibung der Herstellung des entsprechenden Ausgangsstoffes des Beispiels 2 angegebene Weise der aktive Ester hergestellt. Das Produkt wird durch Anreiben mit Diisopropyläther gereinigt. 8,66 g (80,4%) tert.-Butyloxycarbonyl-L-tyrosyl-D-methionyl-glycin-pentachlorphenylester werden erhalten. Schmp.: 175–176 °C; $R_f^{12}$: 0,25–0,35; $[\alpha]_D^{20} = +21{,}7°$ (c = 1, Dimethylformamid).

Beispiel 7

L-Tyrosyl-D-norleucyl-glycyl-L-phenylalanyl-L-α-aminopentanphosphonsäure

Aus 1,08 g (2,4 mMol) tert.-Butyloxycarbonyl-L-tyrosyl-D-norleucyl-glycin und 0,63 g (2 mMol) L-Phenylalanyl-L-α-aminopentanphosphonsäure (Beispiel 1, Schritt 2) wird auf die im Schritt des Beispiels 1 beschriebene Weise das obige Produkt in einer Ausbeute von 0,85 g (65%) erhalten. Schmp.: 196–198 °C; $[\alpha]_D^{20} = +8°$ (c = 1, n Natronlauge); $R_f^5$: 0,35–0,40.

Das als Ausgangsstoff verwendete tert.-Butyloxycarbonyl-L-tyrosyl-D-norleucyl-glycin ist wie folgt beschrieben erhalten worden.

11,57 g (50 mMol) tert.-Butyloxycarbonyl-D-norleucin und 5,6 ml (50 mMol) N-Methylmorpholin werden in 50 ml Dimethylformamid gelöst. Die Lösung wird auf −10 °C gekühlt und unter Rühren mit 6,6 ml (50 mMol) Chlorkohlensäureisobutylester versetzt. Nach 10 Minuten wird das Reaktionsgemisch auf −20 °C gekühlt und die auf −20 °C gekühlte Lösung von 18,6 g (55 mMol) Glycinbenzylester-p-toluol-sulfonat und 6,1 ml (55 mMol) N-Methylmorpholin in 80 ml Dimethylformamid zugesetzt. Das Reaktionsgemisch wird bei −10 °C 10 Minuten lang, dann eine Stunde lang bei 0 °C und schliesslich über Nacht bei

Raumtemperatur gerührt. Dann wird das Reaktionsgemisch filtriert und das Filtrat unter vermindertem Druck eingedampft. Der Rückstand wird in einem Zweiphasensystem aus 300 ml Äthylacetat und 100 ml Wasser gelöst. Die organische Phase wird nacheinander mit 2 × 60 ml 5%iger Natriumhydrogencarbonatlösung, 2 × 60 ml Wasser, 2 × 60 ml eiswassergekühlter n Salzsäure und 2 × 60 ml Wasser gewaschen, über Natriumsulfat getrocknet, mit Aktivkohle geklärt und unter vermindertem Druck eingedampft. Der kristalline Rückstand wird mit Diisopropyläther verrieben, abfiltriert, mit Diisopropyläther gewaschen und dann getrocknet. 14,65 g (76,9%) tert.-Butyloxycarbonyl-D-norleucyl-glycin-benzylester werden erhalten. Schmp.: 85–86 °C; $R_f^{13}$: 0,75–0,85; $[\alpha]_D^{20}$ = +12,2° (c = 1, Dimethylformamid).

Aus 14,2 g (37,5 mMol) des wie vorstehend beschrieben erhaltenen geschützten Dipeptidesters und 21,85 g (41,25 mMol) tert.-Butyloxycarbonyl-L-tyrosin-pentachlorphenylester wird unter Verwendung von 4,2 und 4,6 ml N-Methylmorpholin auf die bei der Beschreibung der Herstellung des Ausgangsstoffes des Beispiels 5 beschriebene Weise tert.-Butyloxycarbonyl-L-tyrosol-D-norleucyl-glycin-benzylester hergestellt. 15,44 g (76%) Produkt werden erhalten. Schmp.: 122 °C; $R_f^{12}$: 0,25–35; $[\alpha]_D^{20}$ = +6° (c = 1, Dimethylformamid).

14,9 g (27,5 mMol) des wie vorstehend beschrieben erhaltenen Tripeptidesters werden in 300 ml Methanol gelöst, die Lösung wird mit 55 ml n Natronlauge versetzt und drei Stunden lang stehen gelassen. Dann wird aus der Lösung unter vermindertem Druck das Methanol abdestilliert, die wässrige Lösung wird mit 3 × 15 ml Äthylacetat ausgeschüttelt, ihr pH-Wert wird mit fester Citronensäure auf 3 eingestellt. Die ausgeschiedene Substanz wird abfiltriert, mit Wasser gewaschen und im Vakuumexsikkator getrocknet. 8,1 g (65%) Produkt werden erhalten. Schmp.: 174–175 °C; $R_f^{2}$: 0,35–0,45; $[\alpha]_D^{20}$ = +2,0° (c = 1, Dimethylformamid).

Beispiel 8

L-Tyrosyl-D-norleucyl-glycyl-L-phenylalanyl-L-α-aminopentansulfonsäure

Schritt 1: tert.-Butyloxycarbonyl-L-tyrosyl-D-norleucyl-glycyl-L-phenylalanyl-L-α-aminopentansulfonsäure-N-methylmorpholinsalz

Aus 2,2 g (3,15 mMol) des aktiven Esters tert.-Butyloxycarbonyl-L-tyrosyl-D-norleucyl-glycin-pentachlorphenylester und 0,95 g (3 mMol) des gemäss Schritt 3 des Beispiels 2 hergestellten Dipeptides werden auf die im Schritt 4 des Beispiels 2 beschriebene Weise 1,63 g (64%) des obigen Produktes erhalten. Schmp.: 134–135 °C; $R_f^{5}$: 0,6–0,7; $[\alpha]_D^{20}$ = −18,8° (c = 1, Dimethylformamid).

Schritt 2: L-Tyrosyl-D-norleucyl-glycyl-L-phenylalanyl-L-α-aminopentansulfonsäure

1,5 g (1,75 mMol) des gemäss dem vorhergehenden Schritt hergestellten Pentapeptidsalzes werden auf die im Schritt 5 des Beispiels 2 beschriebene Weise umgesetzt mit dem Unterschied, dass der Niederschlag auch mit 10 ml Wasser gewaschen wird. 0,91 g (80%) Produkt werden erhalten. Schmp.: 198–200 °C; $R_f^{6}$: 0,33–0,43; $[\alpha]_D^{20}$ = −12,1° (c = 1, Methanol).

Aus 6,8 g (15 mMol) tert.-Butyloxycarbonyl-L-tyrosyl-D-norleucyl-glycin (Beispiel 7, Beschreitung der Herstellung von dessen Ausgangsstoff) wird auf die bei der Beschreibung der Herstellung des entsprechenden Ausgangsstoffes des Beispiels 2 angegebene Weise der aktive Ester hergestellt. Das Endprodukt wird durch Anreiben mit Äther gereinigt. 10,3 g (98%) Produkt werden erhalten. Schmp.: 175–177 °C; $R_f^{12}$: 0,5–0,6.

Beispiel 9

L-Tyrosyl-D-norleucyl-glycyl-L-phenylalanyl-L-α-amino-γ-methylbutansulfonsäure

Schritt 1: Benzyloxycarbonyl-L-phenylalanyl-DL-α-amino-γ-methylbutansulfonsäure

33 g (110 mMol) Benzyloxycarbonyl-L-phenylalanin und 12,2 ml (110 mMol) N-Methylmorpholin werden in 200 ml Dimethylformamid gelöst. Die Lösung wird auf −10 °C gekühlt und bei dieser Temperatur zuerst mit 14,5 ml (110 mMol) Chlorkohlensäureisobutylester, 10 Minuten später mit der Lösung von 16,7 g (100 mMol) DL-α-Amino-γ-methylbutansulfonsäure und 14 ml (100 mMol) Triäthylamin in 200 ml Dimethylformamid versetzt. Das Reaktionsgemisch wird eine Stunde lang bei −10 °C, danach vier Stunden lang bei 0 °C gerührt und dann unter vermindertem Druck eingedampft. Der Rückstand wird in 500 ml Wasser gelöst und die Lösung mit 3 × 100 ml Diäthyläther ausgeschüttelt. Die wässrige Phase wird mit konzentrierter Salzsäure auf pH 2 angesäuert und mit 3 × 100 ml n-Butanol, das mit Wasser gesättigt ist, ausgeschüttelt. Die n-Butanolphasen werden vereinigt und mit 2 × 50 ml butanolgesättigtem Wasser gewaschen. Die Lösung wird unter vermindertem Druck eingedampft. 27 g (85%) Produkt werden erhalten. $R_f^{2}$: 0,20–0,30.

Schritt 2: L-Phenylalanyl-D-α-amino-γ-methylbutansulfonsäure

Das gemäss Schritt 1 erhaltene geschützte Dipeptid (27 g, 85,87 mMol) wird in einem Gemisch aus 600 ml Methanol und 60 ml 25%igem wässrigem Ammoniak gelöst und in Gegenwart von Palladiumaktivkohle hydriert. Nach Ablauf der Reaktion wird der Katalysator abfiltriert und mit 50%igem wässrigem Methanol gewaschen. Das mit der Waschflüssigkeit vereinigte Filtrat wird unter vermindertem Druck eingedampft. Der Rückstand wird im Vakuumexsikkator über konzentrierter Schwefelsäure getrocknet und dann mit 50 ml Wasser einer Temperatur von 60–80 °C verrührt. Die kristallhaltige Suspension wird über Nacht bei etwa 5 °C stehen gelassen, dann filtriert und das Produkt mit 5 ml kaltem Wasser gewaschen. Filtrat und Waschflüssigkeit werden vereinigt und für den Schritt 3 bereitgestellt. Die kristalline Substanz wird im Vakuumexsikkator getrocknet. 10,8 g (34,35 mMol, 80%) Produkt werden erhalten. Schmp.: 254–256 °C; $[\alpha]_D^{20}$ = +127,0° (c = 1, n Natronlauge).

Schritt 3: L-Phenylalanyl-L-α-amino-γ-methylbutansulfonsäure

Die im Schritt 2 bei der Kristallisation angefallene Mutterlauge wird unter vermindertem Druck zur Trockne eingedampft, der Rückstand wird mit Tetrahydrofuran verrührt, abfiltriert, mit Tetrahydrofuran gewaschen und dann getrocknet. 10,1 g (32,2 mMol) Produkt werden erhalten. Schmp.: 226–228 °C; $[\alpha]_D^{20} = -77$ bis $-85°$ (c = 1, n Natronlauge).

Schritt 4: L-Tyrosyl-D-norleucyl-glycyl-L-phenylalanyl-L-α-amino-γ-methylbutansulfonsäure

1,08 g (2,4 mMol) tert.-Butyloxycarbonyl-L-tyrosyl-D-norleucyl-glycin (Beispiel 7, Beschreibung der Herstellung von dessen Ausgangsstoff) und 0,64 g (2 mMol) L-Phenylalanyl-L-α-amino-γ-methylbutansulfonsäure (Beispiel 9, Schritt 3) werden auf die im Schritt 3 des Beispiels 1 beschriebene Weise kondensiert. Nach dem Eindampfen des Reaktionsgemisches wird der Rückstand in 30 ml Wasser gelöst und die Lösung mit 3 × 10 ml Äthylacetat ausgeschüttelt. Die vereinigten organischen Phasen werden mit 2 × 5 ml Wasser gewaschen. Die wässrigen Phasen werden vereinigt, mit n Schwefelsäure auf pH 2 angesäuert und mit 3 × 10 ml eines im Volumverhältnis 2 : 1 bereiteten Gemisches aus Äthylacetat und n-Butanol ausgeschüttelt. Die Äthylacetat-n-Butanol-Lösungen werden vereinigt, mit 2 × 5 ml Wasser, das mit Butanol gesättigt ist, gewaschen und dann unter vermindertem Druck eingedampft. Der Rückstand wird in 10 ml Trifluoressigsäure aufgelöst. Die Lösung lässt man 30 Minuten lang stehen und dampft sie dann unter vermindertem Druck ein. Der Rückstand wird mit Äther verrieben, filtriert, mit Äther gewaschen und dann getrocknet. 0,9 g (80%) Produkt werden erhalten, das bei 199–200 °C schmilzt. $[\alpha]_D^{20} = 1,0°$ (c = 1, n Natronlauge); $R_f^5$: 0,50–0,60.

Die als Ausgangsstoff verwendete DL-α-Amino-γ-methylbutansulfonsäure ist wie folgt beschrieben erhalten worden.

63,5 ml (510 mMol) Isovaleraldehyd werden auf die bei der Beschreibung der Herstellung des entsprechenden Ausgangsstoffes des Beispiels 2 angegebene Weise zu 40 g (47,7%) DL-α-Amino-α-methylbutansulfonsäure umgesetzt. Schmp.: 142–144 °C.

Beispiel 10

L-Tyrosyl-glycyl-glycyl-L-phenylalanyl-D-α-aminopentansulfonsäure

Schritt 1: tert.-Butyloxycarbonyl-L-tyrosyl-glycyl-glycyl-L-phenylalanyl-D-α-aminopentansulfonsäure-N-methylmorpholinsalz

Aus 0,95 g (3 mMol) Dipeptid (Beispiel 2, Schritt 2) und 2,0 g (3,15 mMol) wie bei der Beschreibung der Herstellung des entsprechenden Ausgangsstoffes des Beispieles 2 angegeben erhaltenem tert.-Butyloxycarbonyl-L-tyrosyl-glycyl-glycin-pentachlorphenylester werden auf die im Schritt 4 des Beispiels 2 beschriebene Weise 2,2 g (92,5%) der obigen Verbindung erhalten. Schmp.: 133 °C; $R_f^7$: 0,23–0,33; $[\alpha]_D^{20} = -16,6°$ (c = 1, Dimethylformamid).

Schritt 2: L-Tyrosyl-glycyl-glycyl-L-phenylalanyl-D-α-aminopentansulfonsäure

1,4 g (1,75 mMol) des gemäss Schritt 1 erhaltenen Salzes des geschützten Pentapeptides werden auf die im Schritt 5 des Beispiels 2 beschriebene Weise zu 1,0 g (98%) der obigen Verbindung umgesetzt. Schmp.: 192–194 °C; $R_f^5$: 0,20–0,35; $R_f^8$: 0,35–0,45; $[\alpha]_D^{20} = +6,01°$ (c = 1, 90%ige Essigsäure).

Beispiel 11

L-Tyrosyl-D-alanyl-glycyl-L-phenylalanyl-D-α-aminopentansulfonsäure

Schritt 1: tert.-Butyloxycarbonyl-L-tyrosyl-D-alanyl-glycyl-L-phenylalanyl-D-α-aminopentansulfonsäure-triäthylaminsalz

0,95 g (3 mMol) Dipeptid (Beispiel 2, Schritt 2) und 2,1 g (3,15 mMol) tert.-Butyloxycarbonyl-L-tyrosyl-D-alanyl-glycin-pentachlorphenylester (Beispiel 4, Beschreibung der Herstellung von dessen Ausgangsstoff) werden auf die im Schritt 4 des Beispiels 2 beschriebene Weise kondensiert, wobei jedoch als tertiäre Base 0,4 und 0,45 ml Triäthylamin verwendet werden. 2,40 g (99%) Produkt werden erhalten. Schmp.: 123–124 °C; $R_f^5$: 0,4–0,5; $[\alpha]_D^{20} = -29,9°$ (c = 1, Dimethylformamid).

Schritt 2: L-Tyrosyl-D-alanyl-glycyl-L-phenylalanyl-D-α-aminopentansulfonsäure

1,4 g (1,75 mMol) des gemäss Schritt 1 erhaltenen Pentapeptidsalzes werden auf die im Beispiel 2, Schritt 5, beschriebene Weise umgesetzt mit dem Unterschied, dass der Niederschlag auch noch mit 10 ml Wasser gewaschen wird. 0,78 g (73,3%) Produkt werden erhalten. Schmp.: 262–264 °C; $R_f^7$: 0,3–0,4; $[\alpha]_D^{20} = +25,4°$ (c = 1, 80%ige Essigsäure).

Beispiel 12

L-Tyrosyl-D-methionyl-glycyl-L-phenylalanyl-D-α-aminopentansulfonsäure

Schritt 1: tert.-Butyloxycarbonyl-L-tyrosyl-D-methionyl-glycyl-L-phenylalanyl-D-α-aminopentansulfonsäure-N-methylmorpholinsalz

0,95 g (3 mMol) Dipeptid (Beispiel 2, Schritt 2) und 2,25 g (3,15 mMol) wie bei der Beschreibung der Herstellung des Ausgangsstoffes des Beispiels 6 angegeben erhaltenen tert.-Butyloxycarbonyl-L-tyrosyl-D-methionyl-glycin-pentachlorphenylester werden auf die im Schritt 1 des Beispiels 6 beschriebene Weise kondensiert. 1,96 g (74,6%) Produkt werden erhalten. Schmp.: 125–127 °C; $R_f^5$: 0,45–0,55; $[\alpha]_D^{20} = -14,6°$ (c = 1, Dimethylformamid).

Schritt 2: L-Tyrosyl-D-methionyl-glycyl-L-phenylalanyl-D-α-aminopentansulfonsäure

1,52 g (1,75 mMol) des gemäss Schritt 1 erhaltenen Salzes werden auf die im Schritt 2 des Beispiels 6 beschriebene Weise umgesetzt. 0,9 g (73,3%) Produkt werden erhalten. Schmp.: 262–264 °C; $R_f^6$: 0,1–0,2; $R_f^5$: 0,35–0,45; $[\alpha]_D^{20} = +26,8°$ (c = 1, Trifluoressigsäure).

Beispiel 13

L-Tyrosyl-D-norleucyl-glycyl-L-phenylalanyl-D-α-aminopentansulfonsäure

Schritt 1: tert.-Butyloxycarbonyl-L-tyrosyl-D-norleucyl-glycyl-L-phenylalanyl-D-α-aminopentansulfonsäure-N-menthylmorpholinsalz

0,95 g (3 mMol) Dipeptid (Beispiel 2, Schritt 2) und 2,2 g (3,15 mMol) wie bei der Beschreibung der Herstellung des Austangsstoffes des Beispieles 8 angegeben erhaltener tert.-Butyloxycarbonyl-L-tyrosyl-D-norleucyl-glycin-pentachlorphenylester werden auf die im Schritt 4 des Beispiels 2 beschriebene Weise kondensiert. 1,9 g (75%) Produkt werden erhalten. Schmp.: 132–133 °C; $R_f^5$: 0,7–0,8; $[\alpha]_D^{20}$ = −15,6° (c = 1, Dimethylformamid).

Schritt 2: L-Tyrosyl-D-norleucyl-glycyl-L-phenylalanyl-D-α-aminopentansulfonsäure

1,5 g (1,75 mMol) des gemäss Schritt 1 erhaltenen Salzes werden auf die im Schritt 2 des Beispiels 6 beschriebene Weise umgesetzt. 1,02 g (90%) Produkt werden erhalten. Schmp.: 257 °C; $R_f^6$: 0,4–0,5; $[\alpha]_D^{20}$ = +3° (c = 1, Trifluoressigsäure).

Beispiel 14

L-Tyrosyl-D-methionyl-glycyl-L-phenylalanyl-D-α-aminopentanphosphonsäure

1,13 g (2,4 mMol) tert.-Butyloxycarbonyl-L-tyrosyl-D-methionyl-glycin (Beispiel 5, Beschreibung der Herstellung von dessen Ausgangsstoff) werden mit 0,63 g (2 mMol) L-Phenylalanyl-D-α-aminopentanphosphonsäure (Beispiel 1, Schritt 1 auf die im Schritt 3 des Beispiels 1 beschriebene Weise umgesetzt. 0,9 g (70%) Produkt werden erhalten. Schmp.: 262–265 °C; $[\alpha]_D^{20}$ = +59,2° (c = 1, n Natronlauge); $R_f^5$: 0,35–0,40.

Beispiel 15

L-Tyrosyl-D-norleucyl-glycyl-L-phenylalanyl-D-α-amino-γ-methylbutansulfonsäure

1,08 g (2,4 mMol) tert.-Butyloxycarbonyl-L-tyrosyl-D-norleucyl-glycin (Beispiel 7, Beschreibung der Herstellung von dessen Ausgangsstoff) und 0,64 g (2 mMol) L-Phenylalanyl-D-α-amino-γ-methylbutansulfonsäure (Beispiel 9, Schritt 2) werden auf die im Schritt 4 des Beispiels 9 beschriebene Weise miteinander umgesetzt. 0,95 g (84%) Produkt werden erhalten. Schmp.: 187–190 °C; $[\alpha]_D^{20}$ = +58,9° (c = 1, n Natronlauge).

Beispiel 16

L-Tyrosyl-D-phenylalanyl-glycyl-L-phenylalanyl-L-α-aminopentansulfonsäure

Schritt 1: tert.-Butyloxycarbonyl-L-tyrosyl-D-phenylalanyl-glycyl-L-phenylalanyl-L-α-aminopentansulfonsäure-N-methylmorpholinsalz

2,3 g (3,15 mMol) tert.-Butyloxycarbonyl-L-tyrosyl-D-phenylalanyl-glycin-pentachlorphenylester und 0,95 g (3 mMol) Dipeptid (Beispiel 2, Schritt 3) werden auf die im Schritt 4 des Beispiels 2 beschriebene Weise miteinander umgesetzt. 1,87 g (70,6%) Produkt werden erhalten. Schmp.: 130–132 °C; $R_f^4$: 0,3–0,4; $[\alpha]_D^{20}$ = −12,3° (c = 1, Dimethylformamid).

Schritt 2: L-Tyrosyl-D-phenylalanyl-glycyl-L-phenylalanyl-L-α-aminopentansulfonsäure

1,55 g (1,75 mMol) des gemäss Schritt 1 erhaltenen geschützten Pentapeptides werden auf die im Schritt 5 des Beispiels 2 beschriebene Weise umgesetzt. 1,08 g (90,8%) der obigen Verbindung werden erhalten. Schmp.: 198–204 °C; $R_f^5$: 0,6–0,7; $R_f^8$: 0,08–0,9; $[\alpha]_D^{20}$ = −43° (c = 1, Trifluoressigsäure).

Der als Ausgangsstoff eingesetzte tert.-Butyloxycarbonyl-L-tyrosyl-D-phenylalanyl-glycin-pentachlorphenylester ist wie folgt beschrieben erhalten worden.

13,3 g (50 mMol) tert.-Butyloxycarbonyl-D-phenylalanin und 5,6 ml (50 mMol) N-Methylmorpholin werden in 50 ml Dimethylformamid gelöst. Die Lösung wird auf −10 °C gekühlt und unter Rühren mit 6,6 ml (50 mMol) Chlorkohlensäureisobutylester versetzt. Nach 10 Minuten wird das Reaktionsgemisch auf −20 °C abgekühlt und die auf −20 °C gekühlte Lösung von 18,6 g (55 mMol) Glycin-benzylester-p-toluolsulfonat und 6,1 ml (55 mMol) N-Methylmorpholin in 80 ml Dimethylformamid zugegeben. Das Reaktionsgemisch wird bei −10 °C 10 Minuten lang, bei 0 °C eine Stunde lang und bei Raumtemperatur über Nacht gerührt. Dann wird das Gemisch filtriert und das Filtrat unter vermindertem Druck eingedampft. Der Rückstand wird in einem Zweiphasengemisch aus 300 ml Äthylacetat und 100 ml Wasser gelöst. Die organische Phase wird nacheinander mit 2 × 60 ml 5%iger Natriumhydrogencarbonatlösung, 2 × 60 ml Wasser, 2 × 60 ml eiswassergekühlter n Salzsäure und 2 × 60 ml Wasser gewaschen, über Natriumsulfat getrocknet, mit Aktivkohle geklärt und schliesslich unter vermindertem Druck eingedampft. Der kristalline Rückstand wird in einem im Volumverhältnis 1 : 1 bereiteten Gemisch aus Diäthyläther und n-Hexan suspendiert, abfiltriert, mit dem gleichen Lösungsmittelgemisch gewaschen und im Vakuumexsikkator getrocknet. 18,95 g (91,9%) tert.-Butyloxycarbonyl-D-phenylalanyl-glycin-benzylester werden erhalten. Schmp.: 133–134 °C; $R_f^{14}$: 0,28–0,38; $[\alpha]_D^{20}$ = +8° (c = 1, Dimethylformamid).

18,55 g (45 mMol) des wie vorstehend beschrieben erhaltenen Dipeptidesters werden in 60 ml 11–15% Chlorwasserstoff enthaltendem Äthylacetat gelöst. Nach einer halben Stunde wird die Lösung eingedampft und der Rückstand im Vakuumexsikkator über Kaliumhydroxyd getrocknet. Das erhaltene Produkt ($R_f^2$: 0,4–0,5) wird in 40 ml Dimethylformamid gelöst und die Lösung mit 5,0 ml (45 mMol) N-Methylmorpholin und 26,2 g (49,5 mMol) tert.-Butyloxycarbonyl-L-tyrosin-pentachlorphenylester versetzt. Das Reaktionsgemisch wird 16–20 Stunden lang gerührt. Innerhalb der ersten zwei Stunden werden in fünf Portionen 5,5 ml (49,5 mMol) N-Methylmorpholin zu dem Gemisch gegeben. Dann wird das Reaktionsgemisch unter vermindertem Druck eingedampft, und der Eindampfrückstand wird in einem Zweiphasengemisch aus 300 ml Äthylacetat und 50 ml Wasser gelöst. Die organische Phase wird mit 2 × 50 ml eiswassergekühlter n Salzsäure und dann mit 2 × 50 ml Wasser gewaschen, über Natriumsulfat getrocknet, mit Aktivkohle geklärt und schliesslich

unter vermindertem Druck eingedampft. Der kristalline Rückstand wird in Diisopropyläther suspendiert, abfiltriert, mit Diisopropyläther gewaschen und im Vakuumexsikkator getrocknet. 23,5 g (90,8%) tert.-Butyloxycarbonyl-L-tyrosyl-D-phenylalanyl-glycin-benzylester werden erhalten. Schmp.: 169 °C; $R_f^{10}$: 0,45–0,55; $[\alpha]_D^{20} = +16,3°$ (c = 1, Dimethylformamid).

23,0 g (40 mMol) des wie vorstehend beschrieben erhaltenen tert.-Butyloxycarbonyl-L-tyrosyl-D-phenylalanyl-glycin-benzylesters werden in 400 ml Methanol gelöst und in Gegenwart von Palladiumaktivkohle hydriert. Nach Beendigung der Reaktion wird der Katalysator abfiltriert und mit Methanol gewaschen. Das mit der Waschflüssigkeit vereinigte Filtrat wird unter vermindertem Druck eingedampft. Der Rückstand wird mit Diäthyläther verrieben, abfiltriert, mit Diäthyläther gewaschen und im Vakuumexsikkator getrocknet. 17,45 g (89,8%) tert.-Butyloxycarbonyl-L-tyrosyl-D-phenylalanyl-glycin werden erhalten. Schmp.: 105–110 °C; $R_f^{13}$: 0,2–0,3.

7,3 g (15 mMol) des wie vorstehend beschrieben erhaltenen tert.-Butyloxycarbonyl-L-tyrosyl-D-phenylalanyl-glycines werden auf die im Beispiel 2 bei der Herstellung von dessen entsprechendem Ausgangsstoff angegebene Weise zum tert.-Butyloxycarbonyl-L-tyrosyl-D-phenylalanyl-glycin-pentachlorphenylester umgesetzt. 9,68 g (87,9%) tert.-Butyloxycarbonyl-L-tyrosyl-D-phenylalanyl-glycin-pentachlorphenylester werden erhalten. Schmp.: 209–210 °C; $R_f^{2}$: 0,6–0,7; $[\alpha]_D^{20} = +15,4°$ (c = 1, Dimethylformamid).

Beispiel 17

L-Tyrosyl-D-phenylalanyl-glycyl-L-phenylalanyl-D-α-aminopentansulfonsäure

Schritt 1: tert.-Butyloxycarbonyl-L-tyrosyl-D-phenylalanyl-glycyl-L-phenylalanyl-D-α-aminopentansulfonsäure-N-methylmorpholinsalz

0,95 g (3 mMol) Dipeptid (Beispiel 2, Schritt 2 und 2,3 g (3,15 mMol) tert.-Butyloxycarbonyl-L-tyrosyl-D-phenylalanyl-glycin-pentachlorphenylester (Beispiel 16, Beschreibung der Herstellung von dessen Ausgangsstoff) werden auf die im Schritt 4 des Beispiels 2 beschriebene Weise miteinander umgesetzt. 2,45 g (92,5%) Produkt werden erhalten. Schmp.: 134–135 °C; $R_f^{2}$: 0,27–0,37; $[\alpha]_D^{20} = -6,05°$ (c = 1, Dimethylformamid).

Schritt 2: L-Tyrosyl-D-phenylalanyl-glycyl-L-phenylalanyl-D-α-aminopentansulfonsäure

1,55 g (1,75 mMol) des gemäss Schritt 1 erhaltenen Pentapeptidsalzes werden auf die im Schritt 5 des Beispiels 2 beschriebene Weise umgesetzt. 1,09 g (91,2%) Produkt werden erhalten. Schmp.: 250 °C; $R_f^{5}$: 0,7–0,8; $R_f^{7}$: 0,8–0,9; $[\alpha]_D^{20} = -13,1°$ (c = 1, Trifluoressigsäure).

Beispiel 18

L-Tyrosyl-D-norleucyl-glycyl-L-phenylalanyl-L-α-aminopentanphosphonsäure-kupfer(II)salz

130 mg (0,2 mMol) des gemäss Beispiel 7 erhaltenen Pentapeptides werden in 4 ml 0,1n Natronlauge gelöst. Zu der Lösung wird die Lösung von 60 mg (0,24 mMol) Kupfer(II)sulfat-pentahydrat in 1 ml Wasser gegeben. Der ausgefallene Niederschlag wird abgetrennt, mit 3 × 1 ml Wasser gewaschen und im Vakuumexsikkator getrocknet. 127 mg (90%) Kupfersalz werden erhalten. Schmp.: > 260 °C.

Elementaranalyse für $C_{31}H_{44}O_8N_5PCu$ (M = 709,2):

| | | |
|---|---|---|
| berechnet: | N = 9,87% | Cu = 8,96% |
| gefunden: | N = 9,7 % | Cu = 9,0 %. |

Beispiel 19

L-Tyrosyl-D-norleucyl-glycyl-L-phenylalanyl-L-α-aminopentanphosphonsäure-zinksalz

130 mg (0,2 mMol) des gemäss Beispiel 7 erhaltenen Pentapeptides werden in 4 ml 0,1n Natronlauge gelöst. Zu der Lösung wird die Lösung von 53 mg (0,24 mMol) Zinkacetat-dihydrat in 1 ml Wasser gegeben. Der ausgefallene Niederschlag wird abfiltriert, mit 3 × 1 ml Wasser gewaschen und im Vakuumexsikkator getrocknet. 128 mg (90%) Zinksalz werden erhalten. Schmp.: > 260 °C.

Elementaranalyse für $C_{31}H_{44}O_8N_5PZn$ (M = 711,1):

| | | |
|---|---|---|
| berechnet: | N = 9,85% | Zn = 9,19% |
| gefunden: | N = 9,8 % | Zn = 9,05%. |

Beispiel 20

L-Tyrosyl-D-norleucyl-glycyl-L-phenylalanyl-L-α-aminopentansulfonsäure-zinksalz

130 mg (0,2 mMol) des gemäss Beispiel 8 erhaltenen Pentapeptides werden in 2 ml 0,1n Natronlauge gelöst. Zu der Lösung wird die Lösung von 44 mg (0,2 mMol) Zinkacetat-dihydrat in 1 ml Wasser gegeben. Die Lösung wird auf 0 °C gekühlt, der Niederschlag abfiltriert, mit 3 × 1 ml Eiswasser gewaschen und im Vakuumexsikkator getrocknet. Ausbeute: 116 mg (85%); Schmp.: 215–219 °C.

Elementaranalyse für $C_{31}H_{43}O_8N_5SZn$ (M = 711,15):

| | | |
|---|---|---|
| berechnet: | N = 9,85% | Zn = 9,19% |
| gefunden: | N = 10,0% | Zn = 9,1 %. |

## Patentansprüche

1. Pentapeptide vom Encephalintyp der allgemeinen Formel

$$\text{Tyr–X–Gly–Phe–Y} \qquad \text{I,}$$

worin

Tyr einen L-Tyrosylrest,

Gly einen Rest von Glykokoll,

Phe einen Rest von L-Phenylalanin,

X einen Rest von Glykokoll oder einen Rest einer D-α-Aminocarbonsäure, welcher eine Alkylseitenkette mit 1 bis 4 Kohlenstoffatom(en), eine, gegebenenfalls S-methylsubstituierte, Thioalkylseitenkette mit 1 bis 4 Kohlenstoffatom(en) oder eine Phenylalkylseitenkette mit 1 bis 4 Kohlenstoffatom(en) im Alkylteil aufweist und

Y einen Rest einer L-,D- oder DL-α-Aminophosphonsäure oder L-, D- oder DL-α-Aminosulfonsäure, welcher eine Alkylseitenkette mit 1 bis

4 Kohlenstoffatom(en) aufweist, darstellen, sowie ihre Salze.

2. Pentapeptide nach Anspruch 1, dadurch gekennzeichnet, dass die Alkylseitenkette beziehungsweise Thioalkylseitenkette des Restes der D-α-Aminocarbonsäure, für den X stehen kann, eine solche mit 1, 2 oder 4 Kohlenstoffatom(en) ist.

3. Pentapeptide nach Anspruch 1, dadurch gekennzeichnet, dass die Phenylalkylseitenkette des Restes der D-α-Aminocarbonsäure, für den X stehen kann, eine solche mit 1 oder 2, insbesondere 1, Kohlenstoffatom(en) im Alkylteil ist.

4. Pentapeptide nach Anspruch 1 bis 3, dadurch gekennzeichnet, dass der Rest der D-α-Aminocarbonsäure, für den X stehen kann, ein Rest von D-Alanin, D-Norleucin, D-Methionin oder D-Phenylalanin ist.

5. Pentapeptide nach Anspruch 1 bis 4, dadurch gekennzeichnet, dass die Alkylseitenkette des Restes der L-, D- beziehungsweise DL-α-Aminophosphonsäure beziehungsweise L-, D- beziehungsweise DL-α-Aminosulfonsäure, für den Y steht, ein solcher mit 3 oder 4, insbesondere 4, Kohlenstoffatomen ist.

6. L-Tyrosyl-glycyl-glycyl-L-phenylalanyl-L-α-aminopentanphosphonsäure.

7. L-Tyrosyl-D-alanyl-glycyl-L-phenylalanyl-L-α-aminopentanphosphonsäure.

8. L-Tyrosyl-D-alanyl-glycyl-L-phenylalanyl-DL-α-aminopentansulfonsäure.

9. L-Tyrosyl-D-methionyl-glycyl-L-phenylalanyl-L-α-aminopentanphosphonsäure.

10. L-Tyrosyl-D-norleucyl-glycyl-L-phenylalanyl-L-α-aminopentanphosphonsäure.

11. L-Tyrosyl-D-norleucyl-glycyl-L-phenylalanyl-L-α-aminopentansulfonsäure.

12. Verfahren zur Herstellung der Verbindungen nach Anspruch 1 bis 11, dadurch gekennzeichnet, dass man in in der Peptidchemie an sich bekannter Weise eine L-, D- oder DL-α-Aminophosphonsäure oder L-, D- oder DL-α-Aminosulfonsäure mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat derselben kondensiert und gegebenenfalls mit dem erhaltenen am endständigen Stickstoffatom geschützten Peptidzwischenprodukt und dem beziehungsweise den durch etwaige weitere derartige Kondensationen erhaltenen am endständigen Stickstoffatom geschützten weiteren Peptidzwischenprodukt(en) nach in an sich bekannter Weise erfolgendem Entfernen der Schutzgruppe des endständigen Stickstoffatomes eine weitere beziehungsweise weitere Kondensation(en) mit beziehungsweise jeweils mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat derselben vornimmt, wobei man so viele Kondensationen durchführt, wie es zum Einbau aller gewünschten Aminosäureeinheiten erforderlich ist, sowie danach in an sich bekannter Weise vom erhaltenen geschützten Pentapeptidderivat die Schutzgruppe des endständigen Stickstoffatomes und die etwaige(n) Schutzgruppe(n) von anderen Stickstoffatomen beziehungsweise Sauerstoffatomen entfernt, worauf man gegebenenfalls in an sich bekannter Weise das erhaltene Pentapeptid der allgemeinen Formel in ein Säureadditionssalz desselben überführt beziehungsweise gegebenenfalls das erhaltene Säureadditionssalz des Pentapeptides der allgemeinen Formel in ein anderes Säureadditionssalz oder in das Pentapeptid der allgemeinen Formel überführt.

13. Arzneimittel, gekennzeichnet durch einen Gehalt an 1 oder mehr Verbindungen nach Anspruch 1 bis 11 als Wirkstoff beziehungsweise Wirkstoffen, zweckmässigerweise zusammen mit üblichen pharmazeutischen Konfektionierungsmitteln.

**Claims**

1. Pentapeptides of the encephalin type having the general formula

$$\text{Tyr–X–Gly–Phe–Y} \qquad \text{I},$$

wherein

Tyr represents a L-tyrosyl radical,

Gly a radical of glycine,

Phe a radical of L-phenylalanine,

X a radical of glycine or a radical of a D-α-aminocarboxylic acid which has an alkyl side chain having from 1 to 4 carbon atom(s), a thioalkyl side chain having from 1 to 4 carbon atom(s), optionally S-methylsubstituted, or a phenylalkyl side chain having from 1 to 4 carbon atom(s) in the alkyl part and

Y a radical of a L-, D- or DL-α-aminophosphonic acid or L-, D- or DL-α-aminosulphonic acid which has an alkyl side chain having from 1 to 4 carbon atom(s)

as well as their salts.

2. Pentapeptides according to claim 1, characterized in that the alkyl side chain or thioalkyl side chain, respectively, of the radical of the D-α-aminocarboxylic acid for which X can stand is such having 1, 2 or 4 carbon atom(s).

3. Pentapeptides according to claim 1, characterized in that the phenylalkyl side chain of the radical of D-α-aminocarboxylic acid for which X can stand in such having 1 or 2, particularly 1, carbon atom(s) in the alkyl part.

4. Pentapeptides according to claims 1 to 3, characterized in that the radical of the D-α-aminocarboxylic acid for which X can stand is a radical of D-alanine, D-norleucine, D-methionine or D-phenylalanine.

5. Pentapeptides according th claims 1 to 4, characterized in that the alkyl side chain of the radical of the L-, D- or DL-α-aminophosphonic acid, respectively, or L-, D- or DL-α-aminosulphonic acid, respectively, for which Y stands is such having 3 or 4, particularly 4, carbon atoms.

6. L-tyrosyl-glycyl-glycyl-L-phenylalanyl-L-α-aminopentanphosphonic acid.

7. L-tyrosyl-D-alanyl-glycyl-L-phenylalanyl-L-α-aminopentanphosphonic acid.

8. L-tyrosyl-D-alanyl-glycyl-L-phenylalanyl-DL-α-aminopentansulphonic acid.

9. L-tyrosyl-D-methionyl-glycyl-L-phenylalanyl-L-α-aminopentanphosphonic acid.

10. L-tyrosyl-D-norleucyl-glycyl-L-phenylalanyl-L-α-aminopentanphosphonic acid.

11. L-tyrosyl-D-norleucyl-glycyl-L-phenylalanyl-L-α-aminopentansulphonic acid.

12. A process for the preparation of the compounds according to claims 1 to 11, characterized in that one condenses in a manner known per se in the peptide chemistry L-, D- or DL-α-aminophosphonic acid or L-, D- or DL-α-aminosulphonic acid with the amino acid to be incorporated successively and having on its terminal nitrogen atom a splittable-off protective group and, as far as necessary, on another nitrogen atom being optionally present another splittable-off protective group and/or with the peptide fragment or an ester derivative of it, respectively, successively to be incorporated and having on its terminal nitrogen atom a splittable-off protective group and, as far as necessary, on one or several other nitrogen and/or oxygen atom(s) one or several protective group(s) and, if desired, one carries out with the obtained peptide intermediate protected on the terminal nitrogen atom and with the further peptide intermediate(s) obtained by possible further condensations of this type and protected on the terminal nitrogen atom after having removed the protective group of the terminal nitrogen atom one further condensation or further condensations, respectively, with the amino acid or with the respective amino acid, respectively, successively to be incorporated and having on its terminal nitrogen atom a splittable-off protective group and, as far as necessary, on another nitrogen atom or oxygen atom optionally present another splittable-off protective group and/or with the peptide fragment or an ester derivative of it, respectively, successively to be incorporated and having on its terminal nitrogen atom a splittable-off protective group and, as far as necessary, on one or several other nitrogen and/or oxygen atom(s) optionally present one or several protective group(s) accomplishing as much condensations as are necessary for incorporating all desired amino acid units as well as thereafter in a manner known per se selectively step by step or in one step one removes from the obtained protected pentapeptide derivative the protective group of the terminal nitrogen atom and the possible protective group(s) of other nitrogen atoms or oxygen atoms, respectively, whereafter, if desired, in a manner known per se one converts the obtained pentapeptide of the general formula into an acid addition salt of it or, if desired, the obtained acid addition salt of the pentapeptide of the general formula into another acid addition salt or in the pentapeptide of the general formula, respectively.

13. Medicaments, characterized by a content of 1 or more compounds according to claims 1 to 11 as an active principle or active principles, usefully together with usual pharmaceutical processing agents.

**Revendications**

1. Pentapeptides du type encéphaline de formule générale:

Tyr–X–Gly–Phe–Y (1)

dans laquelle:

Tyr représente un radical L-tyrosyle,

Gly un radical de glycocolle,

Phe un radical de L-phénylalanine,

X un radical de glycocolle ou un radical d'un acide D-α-aminocarboxylique qui présente une chaîne latérale alkyle de 1 à 4 atomes de carbone, une chaîne latérale thioalkyle de 1 à 4 atomes de carbone portant éventuellement un substituant S-méthyle ou une chaîne latérale phénylalkyle contenant 1 à 4 atomes de carbone dans le fragment alkyle et,

Y un radical d'un acide L-, D- ou DL-α-aminophosphonique ou d'un acide L-, D- ou DL-α-aminosulfonique qui présente une chaîne latérale alkyle de 1 à 4 atomes de carbone, ainsi que leurs sels.

2. Pentapeptides selon la revendication 1, caractérisés par le fait que la chaîne latérale alkyle ou la chaîne latérale thioalkyle du radical de l'acide D-α-aminocarboxylique que peut représenter X est une chaîne de 1, 2 ou 4 atomes de carbone.

3. Pentapeptides selon la revendication 1, caractérisés par le fait que la chaîne latérale phénylalkyle du radical de l'acide D-α-aminocarboxylique que peut représenter X est une chaîne contenant 1 ou 2, en particulier 1 atome de carbone dans le fragment alkyle.

4. Pentapeptides selon les revendications 1 à 3, caractérisés par le fait que le radical de l'acide D-α-aminocarboxylique que peut représenter X est un radical de D-alamine, de D-norleucine, de D-méthionine ou de D-phénylalanine.

5. Pentapeptides selon les revendications 1 à 4, caractérisés par le fait que la chaîne latérale alkyle du radical de l'acide L-, D- ou DL-α-aminophosphonique ou de l'acide L-, D- ou DL-α-amino-

sulfonique, que représente Y, est une chaîne de 3 ou 4, en particulier 4 atomes de carbone.

6. Acide L-tyrosyl-glycyl-glycyl-L-phénylalanyl-L-α-aminopentanephosphonique.

7. Acide L-tyrosyl-D-alanyl-glycyl-L-phénylalanyl-L-α-aminopentanephosphonique.

8. Acide L-tyrosyl-D-alanyl-glycyl-L-phénylalanyl-DL-α-aminopentanesulfonique.

9. Acide L-tyrosyl-D-méthionyl-glycyl-L-phénylalanyl-L-α-aminopentanephosphonique.

10. Acide L-tyrosyl-D-norleucyl-glycyl-L-phénylalanyl-L-α-aminopentanephosphonique.

11. Acide L-tyrosyl-D-norleucyl-glycyl-L-phénylalanyl-L-α-aminopentanesulfonique.

12. Procédé de préparation des composés selon les revendications 1 à 11, caractérisé par le fait que, de manière en elle-même connue dans la chimie des peptides, on condense un acide L-, D- ou DL-α-aminophosphonique ou un acide L-, D- ou DL-α-aminosulfonique avec l'aminoacide à incorporer ensuite, présentant sur son atome d'azote terminal un groupe protecteur séparable et, dans la mesure où c'est nécessaire, sur un autre atome d'azote éventuellement présent ou un atome d'oxygène, un autre groupe protecteur séparable, et/ou avec le fragment peptide à incorporer ensuite, présentant sur son atome d'azote terminal un groupe protecteur séparable et, dans la mesure où c'est nécessaire, sur un ou plusieurs autres atomes d'azote et/ou d'oxygène éventuellement présents, un ou plusieurs groupes protecteurs, ou avec un dérivé ester de ce fragment, et qu'éventuellement, avec l'intermédiaire peptide obtenu protégé sur l'atome d'azote terminal et le ou les autres intermédiaires peptides obtenus par d'autres condensations éventuelles de ce genre, protégés sur l'atome d'azote terminal, après avoir éliminé de manière en elle-même connue le groupe protecteur de l'atome d'azote terminal, on effectue une condensation supplémentaire ou des condensations supplémentaires avec, ou chaque fois avec, l'aminoacide à incorporer ensuite, présentant sur son atome d'azote terminal un groupe protecteur séparable et, dans la mesure où c'est nécessaire, sur un autre atome d'azote éventuellement présent ou un atome d'oxygène, un autre groupe protecteur séparable et/ou avec le fragment peptide à incorporer ensuite, présentant sur son atome d'azote terminal un groupe protecteur séparable et, dans la mesure où c'est nécessaire, sur un ou plusieurs atomes d'azote et/ou d'oxygène éventuellement présents, un ou plusieurs groupes protecteurs, ou avec un dérivé ester de ce fragment, en effectuant autant de condensations qu'il est nécessaire pour incorporer toutes les unités aminoacide désirées, et qu'ensuite, de manière en elle-même connue, du dérivé pentapeptide protégé obtenu, on élimine le groupe protecteur de l'atome de carbone terminal et le ou les groupes protecteurs éventuels d'autres atomes d'azote ou d'atomes d'oxygène, après quoi, éventuellement, de manière en elle-même connue, on convertit le pentapeptide de formule générale obtenu en un sel d'addition d'acide de celui-ci, ou éventuellement, on convertit le sel d'addition d'acide obtenu du pentapeptide de formule générale en un autre sel d'addition d'acide ou en le pentapeptide de formule générale.

13. Médicament caractérisé par une teneur en un ou plusieurs composés selon les revendications 1 à 11, comme substance(s) active(s), avantageusement en même temps que des agents de conditionnement pharmaceutiques usuels.